# EUROPÄISCHE PATENTSCHRIFT

(11) **EP 2 410 339 B1**
(45) Veröffentlichungstag und Bekanntmachung des Hinweises auf die Patenterteilung: **25.09.2013**
(21) Anmeldenummer: 11173695.5
(22) Anmeldetag: 12.07.2011
(51) Int. Cl.: G01N 33/84

(54) **Verfahren zum Nachweis der Säureproduktion durch kariogene Bakterien**
Method for the detection of acid production by cariogenic bacteria
Procédé de contrôle de la production d'acide à l'aide de bactéries cariogènes

(30) Priorität: 19.07.2010 EP 10169983
(43) Veröffentlichungstag der Anmeldung: 25.01.2012
(73) Patentinhaber: Ivoclar Vivadent AG, 9494 Schaan (LI)
(72) Erfinder: Bischof, Manuela, 9485 Nendeln (LI); Bolis, Carlo, CH-7206 Igis (CH); Lendenmann, Urs, 9472 Grabs (CH); Rheinberger, Volker, 9490 Vaduz (LI)
(74) Vertreter: UEXKÜLL & STOLBERG

(56) Entgegenhaltungen:
- EP-A1- 1 972 938
- EP-A2- 1 253 425
- US-A1- 2003 113 266
- David G.: "CRT bacteria Kariesrisikotest", , 27. November 2002 (2002-11-27), XP002607748, Schaan, Liechtenstein Gefunden im Internet: URL:http://www.google.de/url?q=http://www. ivoclarvivadent.com/zoolu-website/media/do cument/479/CRT%2Bbacteria&sa=U&ei=mhPuTK23 H8GzhAfHuL3JDA&ved=0CA4QFjAA&usg=AFQjCNGxp UZmO_Khd2UE_OxbWoCxU8TyCQ [gefunden am 2010-11-01]

## Beschreibung

### GEBIET DER ERFINDUNG

Die vorliegende Erfindung betrifft ein Verfahren zur semiquantitativen Bestimmung von kariogenen Bakterien in einer Speichel- und/oder Plaqueprobe, bei dem man die in einer Plaque-oder Speichelprobe enthaltenen Mikroorganismen mit einer C-Quelle in Kontakt bringt, die von den kariogenen Bakterien zu Säure fermentiert wird. Die Mikroorganismen werden anschließend unter Bedingungen inkubiert, die eine selektive Säurebildung durch die kariogenen Bakterien ermöglichen. Die Säurebildung wird anschließend nachgewiesen, indem man mindestens einmal innerhalb eines Zeitraums von 12 Stunden nach Zugabe der C-Quelle den pH-Wert bestimmt, wobei die semiquantitative Bestimmung der kariogenen Bakterien in der Probe durch Vergleich des pH-Werts mit mindestens einem Referenzwert erfolgt. Die Erfindung stellt ferner Kits zur Durchführung solcher Verfahren bereit.

### STAND DER TECHNIK

Der Mundraum des Menschen ist normalerweise von mehr als 800 unterschiedlichen Keimen besiedelt, die in einem natürlichen Gleichgewicht zueinander stehen. Diese Mikroflora, die im Wesentlichen aus Bakterien besteht, umfasst jedoch auch Organismen, die der Gesundheit der Zähne schaden können. Dies betrifft insbesondere Bakterien, die in der Lage sind, Kohlenhydrate aus der Nahrung zu organischen Säuren zu metabolisieren. Die Säure, insbesondere Milchsäure, greift den Zahnschmelz an, was zunächst zu einer Demineralisierung der Zahnoberfläche führt. Durch kontinuierliche Besiedelung der demineralisierten Stellen entstehen somit im Laufe der Zeit Läsionen, die durch den Zahnschmelz auf das Zahnbein und das Zahnmark übergreifen können.

Der überwiegende Teil der für Karies ursächlichen Milchsäure geht auf die metabolische Aktivität der "Mutans-Streptokokken" zurück; diese Gruppe umfasst die Spezies *S*. *mutans, S. sobrinus, S.* rattus *und S. cricetus.* Weiterhin von Bedeutung in diesem Zusammenhang ist die Gruppe der Lactobacillen. Während sich diese Bakterien mit verschiedenen anderen Bakterienarten, die den Mundraum besiedeln, im gesunden Zustand in einem stabilen Gleichgewicht befinden, kann es unter besonderen Bedingungen zu einer verstärkten Vermehrung von Mutans-Streptokokken kommen, wodurch das Risiko, eine Karies zu entwickeln, erheblich ansteigt. Die durch Mutans-Streptokokken verursachte Säurebildung führt zu einem sauren Milieu, das wiederum verstärkt das Wachstum von Lactobacillen fördert. Ein verstärktes Wachstum von Lactobacillen geht mit einer weiteren Absenkung des pH-Werts aufgrund der Säureproduktion durch Lactobacillen einher, wodurch ein Fortschreiten der Erkrankung begünstigt wird.

Es ist gezeigt worden, dass innerhalb weniger Minuten nach Zuführung einer Zuckerlösung der pH-Wert im Plaque durch die fermentative Aktivität der den Mundraum besiedelnden Bakterien stark absinkt. Allerdings konnte in einer klinischen Studie auch gezeigt werden, dass Plaque-Proben aus Kindern, bei denen Karies nachgewiesen wurde, und Plaque-Proben von kariesfreien Kindern ein vergleichbares Potential haben, den pH-Wert im Mundraum zu senken (Minah & Lösche, Infection and Immunity, 17: 43-54, 1977). Dies verweist darauf, dass es im Mundraum des Menschen neben Streptokokken und Lactobacillen eine Reihe weiterer Mikroorganismen gibt, die als metabolische Nebenprodukte zwar Säure ausscheiden, jedoch kein signifikantes Kariesrisiko darstellen. Folglich ist die Messung der gesamten Säureproduktion im Speichel nicht geeignet, die Anzahl von kariogenen Bakterien und somit das Kariesrisiko eines Patienten zu ermitteln. Vielmehr wird dieses Risiko maßgeblich durch die Konzentration der Mutans-Streptokokken und Lactobacillen bestimmt.

Die quantitative oder semiquantitative Bestimmung der Mutans-Streptokokken und/oder Lactobacillen im Plaque oder Speichel kann Aufschluss über das Kariesrisiko eines Patienten geben. Aus diesem Grund sind Testverfahren entwickelt worden, die eine Überprüfung von Plaque- oder Speichelproben im Hinblick auf die Konzentration der Keime durch Ausplattieren auf selektiven Nährböden vorsehen. So basiert das als CRT^{©} bacteria von Ivoclar Vivadent erhältliche Testsystem auf der selektiven Anreicherung von Mutans-Streptokokken bzw. Lactobacillen aus Speichelproben auf Agar-Nährböden. Speichelproben werden jeweils auf Bacitracin-haltigem Mitis-Salivarius-Agar (selektiv für Streptokokken) und parallel dazu auf Rogosa-Agar (selektiv für Lactobacillen) ausgestrichen. Durch Auszählung der auf den Agar-Platten gebildeten Kolonien nach zwei- bis dreitägiger Inkubation kann eine Aussage über das jeweilige Karies-Risiko des Patienten getroffen werden. Ähnliche Systeme sind auch als Dentocult von Orion Diagnostics und als Caricult von OralBiotech erhältlich.

Die Kultivierung von Speichelproben auf Nährböden ist allerdings zeitaufwendig. Eine Auswertung der Ergebnisse kann üblicherweise erst etwa zwei bis drei Tage nach dem Ausplattieren der Speichelprobe erfolgen. Aufgabe der vorliegenden Erfindung ist es daher, ein Verfahren zur Verfügung zu stellen, mit dem man in kurzer Zeit verlässlich und reproduzierbar eine Aussage bezüglich der Anzahl kariogener Bakterien in einer Plaque-oder Speichelprobe treffen kann. Das Verfahren sollte ohne aufwendige apparative Ausstattung durchführbar sein, und das Ergebnis sollte möglichst innerhalb von wenigen Stunden verfügbar sein.

US 2003/113266 und EP 1972938 beschreiben ein Verfahren zur semiquantitativen Bestimmung von kariogenen Bakterien in eines Plaqueprobe.

Erfindungsgemäß wird die obige Aufgabe durch den Gegenstand der vorliegenden Patentansprüche gelöst. Die vorliegende Erfindung stellt in einem ersten Aspekt ein Verfahren für die semiquantitative Bestimmung von Mutans-Streptokokken in einer Plaque- oder Speichelprobe bereit, bei dem man
(a) die in einer Plaque- oder Speichelprobe enthaltenen Mikroorganismen optional aufkonzentriert;
(b) die Mikroorganismen mit einer C-Quelle in Kontakt bringt, die von den kariogenen Bakterien zu Säure fermentiert wird, wobei die C-Quelle Maltotriose ist;
(c) die Mikroorganismen und die C-Quelle unter Bedingungen inkubiert, die eine selektive Säurebildung durch die kariogenen Bakterien ermöglichen;
(d) mindestens einmal innerhalb eines Zeitraums von 12 Stunden nach Zugabe der C-Quelle den pH-Wert bestimmt;

Die vorliegende Erfindung stellt in einem weiteren Aspekt ein Verfahren für die semiquantitative Bestimmung von Lactobacillen in einer Plaque- oder Speichelprobe bereit, bei dem man
(a) die in einer Plaque- oder Speichelprobe enthaltenen Mikroorganismen optional aufkonzentriert;
(b) die Mikroorganismen mit einer C-Quelle in Kontakt bringt, die von den kariogenen Bakterien zu Säure fermentiert wird, wobei die C-Quelle ausgewählt ist aus der Gruppe bestehend aus Mannose und N-Acetylglucosamin;
(c) die Mikroorganismen und die C-Quelle unter Bedingungen inkubiert, die eine selektive Säurebildung durch die kariogenen Bakterien ermöglichen;
(d) mindestens einmal innerhalb eines Zeitraums von 12 Stunden nach Zugabe der C-Quelle den pH-Wert bestimmt.

Die semiquantitative Bestimmung der kariogenen Bakterien in der Probe erfolgt durch Vergleich des in Schritt (d) bestimmten pH-Werts mit einem oder mehreren Referenzwerten. Der Referenzwert oder die Referenzwerte wurde(n) zuvor durch Verwendung einer bekannten Konzentration der entsprechenden kariogenen Bakterien ermittelt.

Das obige Verfahren richtet sich auf die semiquantitative Bestimmung von Bakterien, die anhand ihrer Säurebildung zur Entwicklung einer Karies beitragen (kariogene Bakterien). Eine Quantifizierung oder Semiquantifizierung dieser Bakterien im Speichel oder Plaque ermöglicht daher eine Aussage über das Risiko für die Entwicklung einer Karies. Das Verfahren basiert auf einer im Wesentlichen selektiven Säurebildung durch Bakterien, die mit der Entwicklung und/oder dem Fortschreiten einer Karies im Zusammenhang stehen. Andere Bakterien, die ebenfalls Teil der Mundflora sind, aber nur einen geringen oder keinen Einfluss auf die Entwicklung oder den Verlauf einer Karies haben, sollen hingegen von der Säurebildung weitestgehend ausgeschlossen werden. Kariogene Bakterien, die mit Hilfe des vorliegenden Verfahrens anhand ihrer Säurebildung semiquantitativ erfasst werden sollen, sind Mutans-Streptokokken und/oder Lactobacillen. Für jede dieser Gruppen von Organismen werden vorliegend Bedingungen offenbart, die eine im Wesentlichen selektive Säurebildung ermöglichen.

Das Verfahren der vorliegenden Erfindung verwendet eine Plaque- oder Speichelprobe aus einem Individuum, bei dem die Anzahl der jeweiligen Bakterien in der Mundhöhle semiquantitativ bestimmt werden soll. Üblicherweise wird es sich bei dem Individuum um einen Menschen handeln, wobei die Verwendung des Verfahrens im veterinärmedizinischen Bereich nicht ausgeschlossen ist. Das Verfahren kann bei menschlichen Individuen beliebigen Alters durchgeführt werden. Es ist allerdings in einem besonderen Aspekt der Erfindung bevorzugt, dass das menschliche Individuum ein Kind ist, vorzugsweise ein Kind vor Vollendung des dritten, vierten, fünften, sechsten oder siebten Lebensjahrs. In diesem Alter kann die Etablierung der Mundhygiene speziell auf das jeweilige Risiko des Kindes abgestimmt werden, um auf diese Weise eine Karies wirkungsvoll zu unterbinden.

Eine Speichelprobe kann unter Anwendung herkömmlicher Methoden gewonnen werden. Üblicherweise beträgt das Volumen der Speichelprobe 0,5, 1, 2, 3, 4, 5, 6, 7, 8, 9, 10 oder 15 ml. In einer bevorzugten Ausführungsform handelt es sich bei der Speichelprobe um eine Probe mit einem Volumen von 0,5-5 ml, die unverdünnt in das erfindungsgemäße Verfahren eingesetzt wird. In einer weiteren bevorzugten Ausführungsform handelt es sich bei der Speichelprobe um eine aufkonzentrierte Probe, d.h. die im Speichel vorhandenen Feststoffe, wie z.B. bakterielle Zellen, wurden durch Filtration, Zentrifugation oder ähnliche Verfahren vom flüssigen Bestandteil des Speichels abgetrennt und in ein kleineres Volumen Flüssigkeit (z.B. Puffer) überführt.

Aus dem Stand der Technik sind mehrere Methoden bzw. Vorrichtungen zur Gewinnung von Speichel bekannt. In einer einfachen Ausführungsform lässt der Spender den Speichel aus der Mundhöhle heraustropfen, wobei dieser in einem geeigneten Probengefäß gesammelt wird. Alternativ dazu kann der Speichel auch mit Hilfe eines Aspirators aus dem Mundraum abgesaugt werden. Sofern größere Volumina Speichel für die Durchführung der erfindungsgemäßen Verfahren gewünscht sind, kann die Speichelbildung mechanisch stimuliert werden, vorzugsweise durch Kaubewegungen. Beispielsweise kann dem Spender ein Paraffinpellet verabreicht werden, das beim Zerkauen die Speichelbildung fördert. Der Speichel kann nach etwa 0,5 bis 5 Minuten gesammelt werden, z.B. nach 2, 3, 4 oder 5 Minuten.

Der aus dem Spender gewonnene Speichel kann in dem Verfahren der vorliegenden Erfindung bei Bedarf auch verdünnt eingesetzt werden. Eine Verdünnung der Speichelprobe kann z.B. erforderlich sein, wenn aufgrund einer zu hohen Viskosität des Speichels eine Aufkonzentrierung mittels Filtration nicht möglich ist oder einen zu langen Zeitraum benötigt. Sofern eine Verdünnung unter den gegebenen Bedingungen angezeigt ist, kann Wasser (z.B. steriles Wasser) oder ein im Bereich der Mikrobiologie üblicher Puffer verwendet werden, der die nachfolgende Fermentation der kariogenen Bakterien nicht wesentlich beeinträchtigt. Geeignete Puffer sind z.B. Tris-Puffer, HEPES-Puffer oder Phosphat-Puffer.

Das erfindungsgemäße Verfahren kann darüber hinaus auch mit Plaqueproben aus dem zu untersuchenden Spender durchgeführt werden. Zu diesem Zweck können Beläge von der Zahnoberfläche mit einem sterilen Bürstchen oder mit einem Spatel durch Abrieb gewonnen und in ein geeignetes Probengefäß überführt werden. Die Plaqueprobe kann anschließend in Wasser oder in einen geeigneten Puffer aufgenommen werden, z.B. in Tris-Puffer, HEPES-Puffer oder Phosphat-Puffer. Die resultierende Bakteriensuspension kann anschließend direkt in das erfindungsgemäße Verfahren eingesetzt werden.

In Abhängigkeit von den nachzuweisenden kariogenen Bakterien kann es in einem ersten Schritt des erfindungsgemäßen Verfahrens notwendig oder sinnvoll sein, vor Zugabe der Kohlenstoff-quelle zu den Mikroorganismen zunächst eine Aufkonzentrierung der in der Plaque- oder Speichelprobe vorhandenen Mikroorganismen durchzuführen. Eine solche Aufkonzentrierung kann beispielsweise mittels Zentrifugation der vom Spender erhaltenen Plaque- oder Speichelprobe und anschließender Resuspension der in der Probe enthaltenen Feststoffe in einem kleineren Volumen Wasser oder Puffer (z.B. Tris-Puffer, HEPES-Puffer oder Phosphat-Puffer) erreicht werden. Auf diese Weise können mehrere ml Speichel in einem Volumen von 500 µl oder weniger Puffer oder Wasser aufgenommen werden. Dabei können Volumen von weniger als 400 µl, weniger als 300 µl, weniger als 200 µl, weniger als 100 µl, weniger als 50 µl, weniger als 25 µl, weniger als 20 µl, weniger als 15 µl, weniger als 10 µl, weniger als 5 µl oder weniger als 1 µl verwendet werden. Die nach Zentrifugation erhaltenden Feststoffe können auch direkt in einem Teil des Überstands resuspendiert werden.

Eine Aufkonzentrierung der erfindungsgemäß gewonnenen Plaque-oder Speichelprobe kann darüber hinaus auch durch Filtration erfolgen. Dabei können Filter eingesetzt werden, die aus der mikrobiologischen Praxis bekannt sind, z.B. Membranen, die für die Sterilfiltration von Lösungen verwendet werden. Diese Filter weisen eine Porengröße auf, die gering genug ist, um den Durchtritt von Bakterien aus der Speichelprobe in das Permeat zu verhindern. Es ist zu beachten, dass Streptokokken im Speichel in der Regel in Form von Biofilmen wachsen und folglich in einer Matrix aus extrazellulären Polysacchariden vorliegen. Aus diesem Grund können Streptokokken auch mit Filtern mit einer Porengröße von etwa 15 µm oder mehr wirksam aus dem Speichel filtriert werden. Geeignete Filter für Lactobacillen weisen vorzugsweise eine Porengröße von weniger als 2 µm, weniger als 1 µm, weniger als 0,5 µm, weniger als 0,3 µm auf, z.B. weniger als 0,25 µm. In einer erfindungsgemäß besonders bevorzugten Ausführungsform weist der Filter für den Einsatz bei der Aufkonzentrierung der Speichelprobe eine Porengröße im Bereich von 0,2-0,22 µm auf. Filter mit derartig kleinen Porengrößen erfordern das Anlegen eines Drucks bei der Filtration. Sofern die Filtration mit einem Filtermaterial durchgeführt wird, das auf einem Saugvlies angeordnet ist (siehe unten) oder lediglich durch die Schwerkraft vermittelt wird, dann müssen entsprechend größere Porenvolumen gewählt werden. Geeignete Porengrößen liegen im Bereich von 1-10 µm, vorzugsweise 2-8 µm.

Für die Filtration im Rahmen der vorliegenden Erfindung werden Filter eingesetzt, die üblicherweise aus polymeren Materialien wie Polypropylen (PP), Polyvinylidenflourid (PVDF), Polytetrafluorethylen (PTFE), Polyethersulfon (PES), Polyvinylpyrrolidon (PVP) oder Polyetheretherketon (PEEK) bestehen. Auch Filter aus Nylon, Cellulose, Nitrocellulose oder aus Glas- oder Quarzfasern finden häufig bei der Abtrennung von bakteriellen Zellen aus Flüssigkeiten Verwendung. Geeignete Filter sind von verschiedenen Herstellern erhältlich, z.B. von Sartorius AG (Göttingen, Deutschland), Millipore (Schwalbach, Deutschland), Whatman (Maidstone, England) oder Pall GmbH (Dreieich, Deutschland). Ein besonders bevorzugtes Filtermaterial ist das Glasfaserfiltermaterial 934-AH von Whatman.

In einer Ausführungsform der vorliegenden Erfindung erfolgt die Filtration mit Hilfe eines mehrlagigen Filters, vorzugsweise eines doppellagigen Filters. Dabei können die verschiedenen Filtermembranen, welche jeweils eine Lage des mehrlagigen Filters bilden, identische oder abweichende Porengrößen aufweisen. Vorzugsweise unterscheiden sich die verschiedenen Lagen hinsichtlich ihrer Porengröße, wobei die Lage des Filters, die als erste in Kontakt mit der Speichelprobe kommt eine größere Porengröße aufweist als die darunter angeordneten Filterlagen. Durch eine solche Anordnung von Filtermembranen kann eine Vorfiltration erreicht werden. So kann in einem doppellagigen Filter z.B. eine erste Membran mit einer Porengröße von 0,4-20 µm vorgesehen sein, unter der eine weitere Membran mit einer Porengröße von 0,2 µm angeordnet ist. Die Oberfläche der Membranen kann bei Bedarf chemisch modifiziert werden, z.B. um die hydrophilen Eigenschaften der Membran zu modifizieren.

Die Filtration kann beispielsweise mit Hilfe einer Spritze erfolgen. Dabei kann ein Aufsatz verwendet werden, der einen geeigneten Filter umfasst, wobei die verdünnte oder unverdünnte Speichelprobe unter Anlegen eines geringen Drucks durch das Filtermaterial geleitet wird. In einer weiteren einfachen Ausführungsform wird unter dem Filter ein Saugvlies angeordnet, das in Kontakt mit dem Filter steht. Bei Auftropfen der verdünnten oder unverdünnten Speichelprobe reichern sich die in der Probe enthaltenen Feststoffe, wie z.B. die bakteriellen Zellen, auf dem Filtermaterial an, während der wässrige Bestandteil der Probe in das Saugvlies geleitet wird. Als Saugvlies können insbesondere Superabsorbermaterialien verwendet werden, wie z.B. die von BASF erhältlichen LUQUASORB-Polymere. Besonders geeignet sind auch Superabsorbermaterialien, die bereits in Vliesform vorliegen, wie z.B. LUQUAFLEECE von BASF. Um das Auftropfen der Proben zu erleichtern können Schablonen verwendet werden, die aus Kunststoff oder Plexiglas gefertigt sind und das gezielte Auftropfen der Plaque- oder Speichelprobe erleichtern. Nach erfolgter Filtration kann das Filtermaterial für die nachfolgenden Schritte von dem Saugvlies getrennt werden.

Die auf dem Filtermaterial verbliebenen Mikroorganismen, insbesondere die kariogenen Bakterien, können vor der Inkubation mit der C-Quelle mit einem Volumen einer geeigneten Flüssigkeit (z.B. Wasser, Tris-Puffer, HEPES-Puffer, Phosphat-Puffer) von dem Filtermaterial gespült werden. Das für das Ablösen von der Membran verwendete Volumen ist dabei geringer als das Ausgangsvolumen der Speichelprobe. Das Volumen der Bakteriensuspension, die im Folgenden für die Inkubation mit der C-Quelle verwendet wird, richtet sich im Wesentlichen nach dem jeweiligen Format des nachfolgenden Säuretests (siehe unten). Sofern z.B. die Inkubation mit der C-Quelle und/oder der Nachweis der Säurebildung in Röhrchen oder Küvetten (z.B. aus Glas oder Kunststoff) durchgeführt werden, kann das Volumen der Bakteriensuspension mehrere ml betragen. Sofern die Inkubation mit der C-Quelle und/oder der Nachweis der Säurebildung auf einem Filter oder auf einer Membran durchgeführt werden (z.B. auf einem wie nachstehend beschriebenen Teststreifen), so wird das Volumen der Bakteriensuspension in der Regel im Bereich von 0,5 bis 2000 µl liegen, z.B. bei etwa 5, 10, 20, 50, 60, 70, 80, 90, 100, 150, 200, 250, 300, 350, 400, 450 oder 500 µl.

In einer besonders bevorzugten Ausführungsform der Erfindung werden die Mikroorganismen, insbesondere die kariogenen Bakterien, nicht von dem Filtermaterial entfernt, sondern verbleiben auf dem für die Aufkonzentrierung verwendeten Filtermaterial und werden direkt auf diesem mit der C-Quelle und/oder den entsprechenden Testreagenzien, z.B. mit einem pH-Indikator, etc., in Kontakt gebracht. Die Säurebildung kann dabei nach Zugabe der C-Quelle und der Testreagenzien z.B. direkt anhand der Veränderung eines pH-Farbindikators auf dem Filtermaterial nachgewiesen werden. Da die Aufnahmefähigkeit der Filter in der Regel begrenzt ist, werden die C-Quelle und Testreagenzien je nach verwendetem Filter in geringen Volumina auf den Filter aufgetragen, vorzugsweise in einem Volumen von etwa 5, 10, 20, 50, 60, 70, 80, 90, 100, 150, 200, 250, 300, 350, 400, 450 oder 500 µl. Besonders bevorzugt ist es, dass das Volumen pro Filterfläche im Bereich von etwa 0,1-15 µl pro mm² Filtermaterial beträgt, vorzugsweise 0,5-10 µl pro mm² Filtermaterial. Die Konzentration der Testreagenzien wird auf das jeweils aufzutragende Volumen abgestellt sein.

Die Aufkonzentrierung, z.B. mittels Filtration, kann bei einer Temperatur von 20-40°C durchgeführt werden, wobei diese in der Regel bei Raumtemperatur (d.h. bei etwa 20°C) durchgeführt werden wird, insbesondere wenn das erfindungsgemäße Verfahren als Schnelltest ausgestaltet ist, der eine Bestimmung der jeweiligen Organismen innerhalb von wenigen Stunden ermöglicht (z.B. im Rahmen einer zahnärztlichen Untersuchung). Da die in der aufkonzentrierten Plaque- oder Speichelprobe enthaltenen kariogenen Bakterien im nachfolgenden Schritt hinsichtlich ihrer Säurebildung untersucht werden sollen, ist zu beachten, dass der Zeitraum zwischen Aufkonzentrierung und Zugabe der Kohlenstoff-Quelle nicht zu lang ist. Dadurch wird gewährleistet dass die in der aufkonzentrierten Probe enthaltenen Bakterien lebensfähig und metabolisch aktiv bleiben. Es ist bevorzugt, dass die aufkonzentrierten kariogenen Bakterien innerhalb von Minuten in die nachfolgende Untersuchung der Säurebildung eingesetzt werden. Vorzugsweise erfolgt die Untersuchung der Säurebildung innerhalb von 1, 5, 10, 15, 20, 25, 30, 60 Minuten nach Aufkonzentrierung der Speichelprobe.

Die Aufkonzentrierung der Plaque- oder Speichelprobe stellt keinen obligatorischen Schritt des erfindungsgemäßen Verfahrens dar. Die Verwendung einer aufkonzentrierten BakterienSuspension hat jedoch einen signifikanten Einfluss auf die Gesamtdauer des Verfahrens bis zur Auswertung. Aufkonzentrierte Proben erlauben eine semiquantitative Auswertung des Verfahrens bereits nach wenigen Stunden, vorzugsweise innerhalb von weniger als drei Stunden, weniger als zwei Stunden, weniger als einer Stunde, z.B. innerhalb von 50 Minuten, innerhalb von 40 Minuten oder innerhalb von 30 Minuten oder weniger. In einer besonders bevorzugten Ausführungsform ist das Verfahren als Schnelltest ausgestaltet, der eine semiquantitative Bestimmung der Konzentration von kariogenen Bakterien in einer Speichelprobe innerhalb von einer Stunde ermöglicht. Dies ermöglicht eine solche Bestimmung bereits im Rahmen eines einzigen Zahnarztbesuchs.

Im nächsten Schritt des Verfahrens werden die in der verdünnten oder unverdünnten Plaque- oder Speichelprobe oder die in der durch Aufkonzentrieren hergestellten Bakteriensuspension enthaltenen Mikroorganismen mit einer Kohlenstoff-Quelle (C-Quelle) in Kontakt gebracht, die von den zu quantifizierenden kariogenen Bakterien zu Säure fermentiert werden kann. Das Verfahren der vorliegenden Erfindung basiert auf dem Anlegen von Bedingungen, unter denen im Wesentlichen nur die nachzuweisenden kariogenen Bakterien zur Bildung von Säure in der Lage sind. Daher ist es besonders bevorzugt, eine C-Quelle auszuwählen, die ausschließlich von den zu quantifizierenden kariogenen Bakterien verwertet werden kann, oder die von diesen Bakterien schneller zu Säure fermentiert werden kann als von anderen Mikroorganismen der Mundflora. Wenn das erfindungsgemäße Verfahren z.B. innerhalb von 1-2 Stunden durchgeführt werden soll, kann es sich als ausreichend erweisen, wenn die zu quantifizierenden kariogenen Bakterien bereits innerhalb von etwa einer Stunde eine nachweisbare Säurebildung zeigen, während die Säurebildung bei anderen in der Speichelprobe vorhandenen Bakterien erst nach 3 Stunden oder später einsetzt, d.h. zu einem Zeitpunkt, an dem das Verfahren bereits abgeschlossen ist.

Im Rahmen der vorliegenden Erfindung ist festgestellt worden, dass unter Bedingungen, die eine Säurebildung durch kariogenen Bakterien stark begünstigen, die Abnahmerate des pH-Werts, d.h. die Abnahme des pH-Werts pro Zeiteinheit, mit der Konzentration der jeweiligen kariogenen Bakterien in der Speichelprobe korreliert ist. Daher kann unter diesen Bedingungen die Messung des pH-Werts zu verschiedenen Zeitpunkten nach Zugabe der C-Quelle Aufschluss über die Anzahl der risikorelevanten kariogenen Bakterien in der in der ursprünglichen Plaque- oder Speichelprobe geben (siehe Beispiele).

Kariogene Bakterien, die für eine Risikobewertung anhand des erfindungsgemäßen Verfahrens besonders geeignet sind, umfassen die Gruppe der sogenannten "Mutans-Streptokokken", welche wiederum die Spezies *S*. *mutans, S. sobrinus, S. rattus und S. cricetus* umfasst. Diese Streptokokken zählen zu den ersten Mikroorganismen, die eine gereinigte Zahnoberfläche neu besiedeln. Sie sind ferner in der Lage, aus Nahrungsresten und Speichelkomponenten einen polymeren Schleim zu bilden, mit dem sie an der Zahnoberfläche anhaften können. Der von den Streptokokken gebildete Biofilm wird auch als Plaque bezeichnet. Es handelt sich um einen strukturierten Belag aus lebenden und toten Mikroorganismen in einer Matrix aus Polysacchariden und Glycoproteinen. Besonders leicht lagert sich Plaque in den Vertiefungen der Kauflächen, im Bereich zwischen den Zähnen und am Zahnfleischrand an. Dieser Biofilm ermöglicht es auch anderen Bakterien, sich auf der Oberfläche des Zahns niederzulassen.

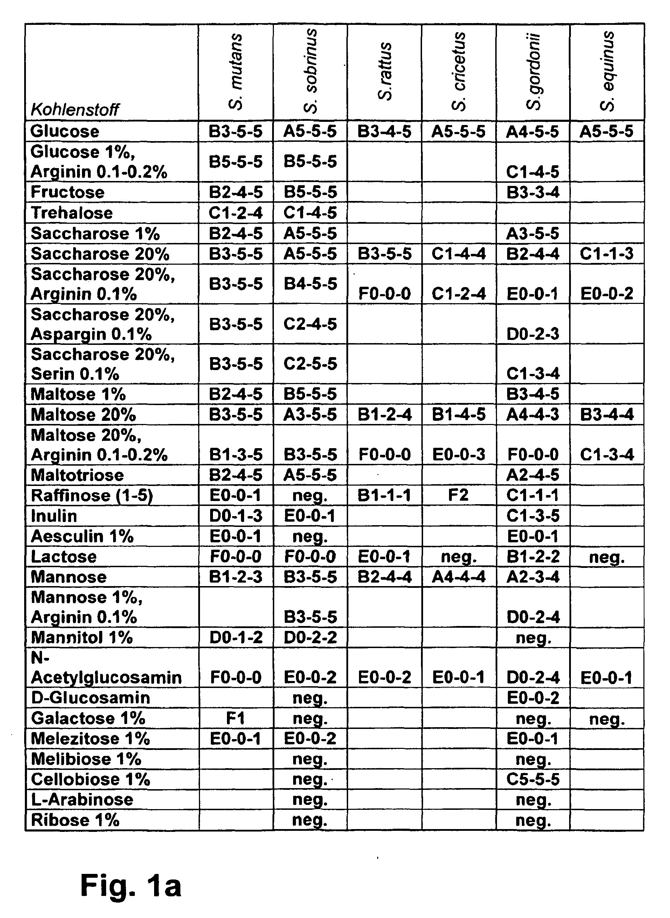

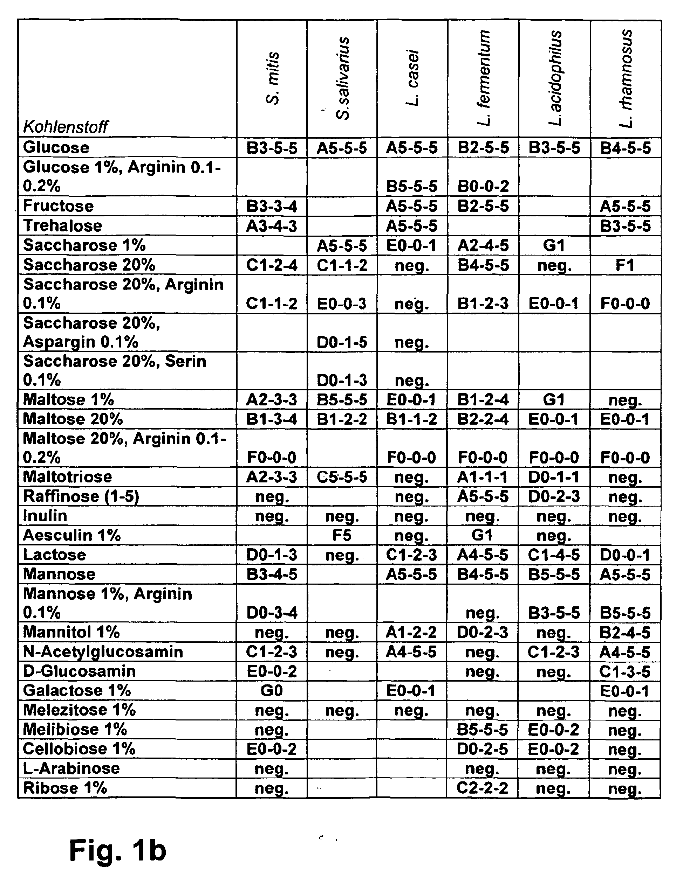

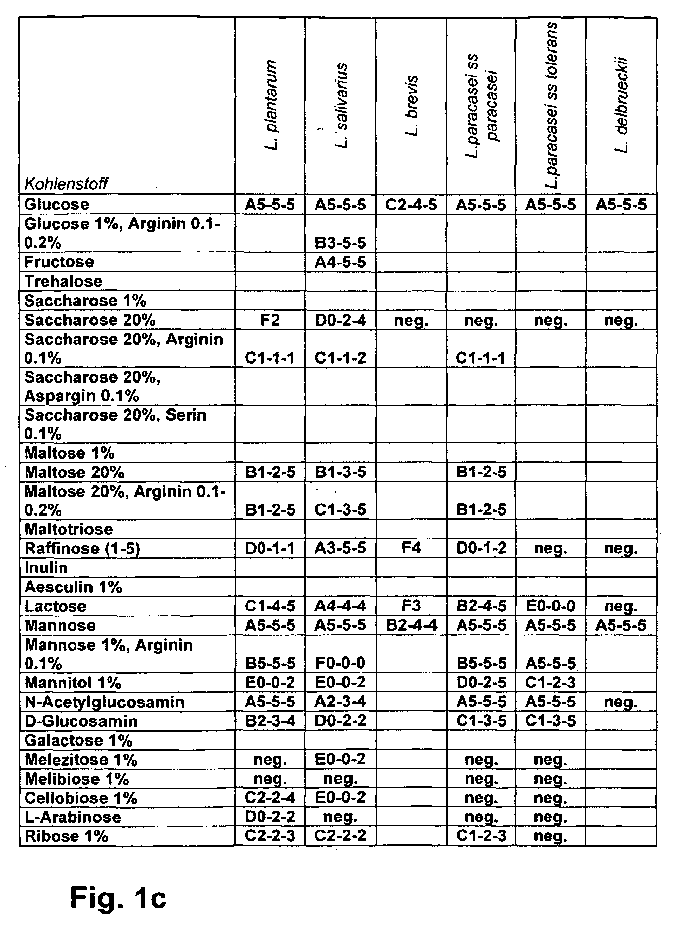

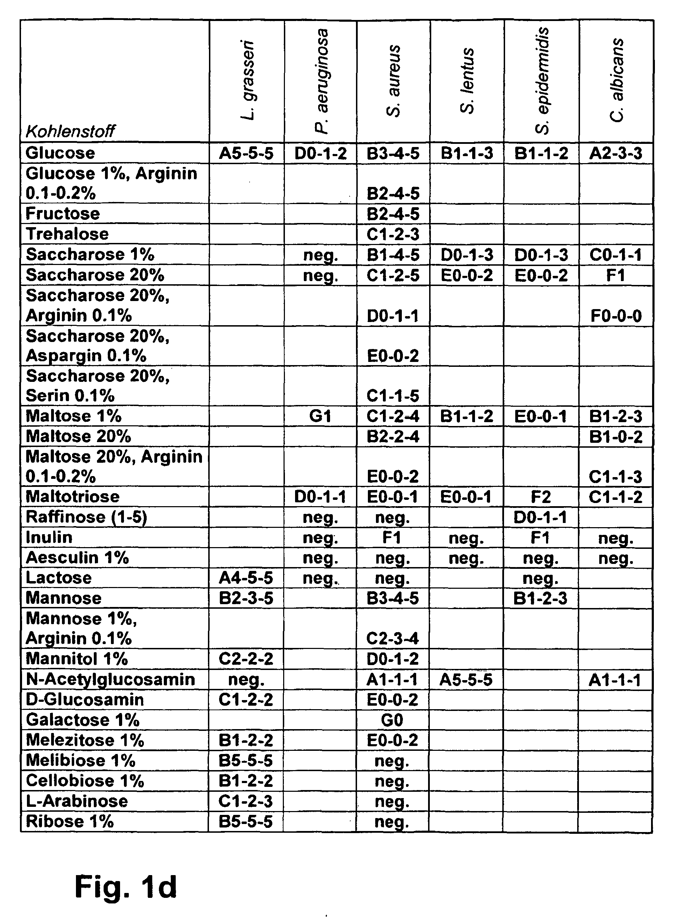

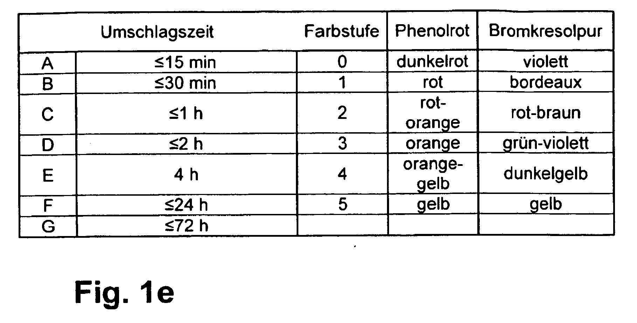

Die Anzahl von Mutans-Streptokokken in einer Speichelprobe ist in besonderer Weise dazu geeignet, auf das Kariesrisiko zu schließen. In dem erfindungsgemäßen Verfahren kann die gewählte C-Quelle von mehreren oder allen der oben genannten Spezies der Mutans-Streptokokken verwertet werden, während andere in der Speichelprobe vorhandenen Bakterien nicht oder nur mit deutlicher Verzögerung in der Lage sind, die C-Quelle zu verwerten, wobei die C-Quelle Maltotriose ist, die von allen Mutans-Streptokokken schnell metabolisiert werden. Maltotriose wird zwar auch von anderen Mikroorganismen im Speichel umgesetzt, jedoch wesentlich langsamer (siehe Figur 1). In einer alternativen Ausführungsform der Beschreibung wird die C-Quelle in dem bestimmten Zeitfenster, in dem das Verfahren durchgeführt wird, nur von einem einzelnen Mitglied der Gruppe der Mutans-Streptokokken verwertet, z.B. von *S*. *sobrinus.* So lässt sich der Tabelle in Figur 1 entnehmen, dass bei Verwendung von Saccharose als C-Quelle in einer Konzentration von 20% im Reaktionsansatz eine Säurebildung innerhalb von 15 Minuten nur bei *S. sobrinus* nachweisen lässt. Wird das erfindungsgemäße Verfahren unter Verwendung dieser C-Quelle durchgeführt, so könnte eine ausschließlich durch diese Spezies verursachte nachgewiesen werden, wenn das Verfahren nach etwa 15-20 Minuten beendet wird, d.h. noch bevor eine Fermentierung der Saccharose durch andere kariogene oder nicht-kariogene Bakterien erfolgt.

In einer alternativen Ausführungsform der vorliegenden Erfindung handelt es sich bei den zu quantifizierenden kariogenen Bakterien um die Gruppe der Lactobacillen, welche die Spezies *L. acidophilus, L. casei, L. rhamnosus, L. brevis, L. plantarum und L. gasseri* umfasst. Bakterien der Gattung Lactobacillus sind zur gemischten Säuregärung fähig, und sie sind darüber hinaus für einen großen Teil der im Mund durch Mikroorganismen gebildeten Säure verantwortlich. Lactobacillen sind insbesondere in der Lage, sich in dem von Streptokokken gebildeten Biofilm anzusiedeln. An einer aktiven Kariesstelle lassen sich immer Stämme der Gattung Lactobacillus identifizieren. Aus den oben genannten Gründen steht auch der quantitative Nachweis von Bakterien der Gattung Lactobacillus im Speichel und in der Plaque gegenwärtig im Zentrum der präventionsorientierten Praxis und Forschung. Kohlenstoffquellen, die eingesetzt werden um eine selektive Säurebildung durch Lactobacillen nachzuweisen, sind N-Acetylglucosamin und Mannose (oder Mischungen derselben).

Die C-Quelle kann dem Reaktionsansatz (bestehend aus den Mikroorganismen der Speichelprobe, der C-Quelle und den weiteren optionalen Bestandteilen) in einer Menge von 0,05 bis 60 Gew.-% zugesetzt werden, z.B. in einer Menge von 0,1%, 0,5%, 1%, 2%, 3%, 4%, 5%, 6%, 7%, 8%, 9%, 10%, 15%, 20%, 30%, 40%, 50%, oder mehr. So hat sich gezeigt, dass z.B. durch Erhöhung der Saccharose-Konzentration in den Testlösungen die Spezifität für bestimmte Mutans-Streptokokken verbessert werden kann.

Der Kontakt zwischen den kariogenen Bakterien der Plaque- oder Speichelprobe und der ausgewählten C-Quelle kann auf mehrere Arten erfolgen. Beispielsweise kann die C-Quelle als Lösung vorliegen, z.B. gelöst in Wasser oder in einem physiologisch verträglichen Puffer. Diese Lösung kann mit einem definierten Volumen einer verdünnten, unverdünnten oder aufkonzentrierten flüssigen Plaque- oder Speichelprobe vermischt werden. Die C-Quelle kann darüber hinaus auch in Form eines Pulvers oder Granulats verwendet werden, das in der verdünnten, unverdünnten oder aufkonzentrierten flüssigen Speichelprobe aufgelöst wird. So können z.B. Röhrchen, Mikrotiterplatten oder Küvetten bereitgestellt werden, die eine definierte Menge der C-Quelle in Pulverform enthalten, sodass bei Zugabe einer flüssigen Speichelprobe der Kontakt zwischen den kariogenen Bakterien und der C-Quelle hergestellt wird. Geeignete C-Quellen, wie z.B. Mannose, sind von verschiedenen Anbietern in Pulverform erhältlich.

In einer bevorzugten Ausführungsform werden die Inkubation mit der C-Quelle sowie die anschließende Bestimmung des pH-Werts in einem flüssigen Reaktionsansatz (Lösung bzw. Suspension) durchgeführt. Abhängig von den in dem Verfahren eingesetzten Volumina von Bakteriensuspension und Testreagenzien können dabei Röhrchen oder Küvetten aus Glas oder Kunststoff, z.B. solche mit einem Volumen von 5-10 ml, Eppendorf-Reaktionsgefäße (mit einem Volumen von 1-2 ml) oder Mikrotiterplatten eingesetzt werden.

In einer weiteren bevorzugten Ausführungsform können die C-Quelle und/oder die Testreagenzien in trockener Form auf einem Filtermaterial, z.B. auf der Oberfläche eines Teststreifens, immobilisiert werden. Als Teststreifen können dabei die oben im Zusammenhang mit der Aufkonzentrierung erwähnten Filtermaterialen Verwendung finden, d.h. Filter deren Porengröße einen Übergang von kariogenen Bakterien, die auf das Filtermaterial aufgetragen werden, in das Permeat verhindern. Die verdünnte, unverdünnte oder aufkonzentrierte flüssige Speichelprobe wird auf das Filtermaterial aufgetropft, wodurch die immobilisierte C-Quelle gelöst und damit für die kariogenen Bakterien zugänglich wird. Die Verwertung der C-Quelle und die daraus resultierende Säurebildung kann unmittelbar auf dem Filtermaterial nachgewiesen werden, z.B. durch Verwendung eines pH-Farbindikators. In einer Ausführungsform der Erfindung handelt es sich bei der C-Quelle um Maltotriose, die immobilisiert auf einem Teststreifen vorliegt. Ein solcher Teststreifen lässt sich zur Bestimmung der Säurebildung durch Mutans-Streptokokken verwenden. In einer alternativen Ausführungsform handelt es sich bei der C-Quelle um N-Acetylglucosamin und/oder Mannose, die immobilisiert auf einem Teststreifen vorliegt. Ein solcher Teststreifen lässt sich zur Bestimmung der Säurebildung durch Lactobacillen verwenden.

In einer weiteren, besonders bevorzugten Ausführungsform handelt es sich bei dem Material, auf dem die C-Quelle und/oder die Testreagenzien in trockener Form immobilisiert werden, um dasselbe Material, das zuvor für die Filtration der Speichelprobe zum Zwecke der Aufkonzentrierung der Speichelprobe verwendet wurde. Dies bedeutet, dass das Filtermaterial zunächst zur Anreicherung von kariogenen Bakterien aus dem Speichel durch Filtration einer Speichelprobe verwendet wird. Bei einer solchen Ausführungsform muss allerdings darauf geachtet werden, dass sich bei der Filtration die immobilisierte C-Quelle und/oder die Testreagenzien nicht oder nur in geringem Umfang von dem Filtermaterial lösen, da sie ansonsten durch den Filter in das Permeat übertreten würden und folglich für den anschließenden Nachweis der Säurereaktion auf dem Filter nicht mehr zur Verfügung stünden. Erst nach Abschluss der Aufkonzentrierung der Plaque- oder Speichelprobe sollte es zu einer Freisetzung der C-Quelle und/oder der Testreagenzien auf dem Filter kommen, sodass die Säurebildung auf dem Filtermaterial stattfinden kann. Dies kann z.B. durch Mittel erreicht werden, die einen zeitlich verzögerten Übergang der immobilisierten Reagenzien in Lösung gewährleisten. So hat es sich als vorteilhaft erwiesen, die C-Quelle und/oder die Testreagenzien (z.B. den Farbindikator) in trockener Form auf ein Filtermaterial aufzutragen und mit einem Lack zu überziehen, der eine verzögerte Freisetzung bewirkt.

Dabei sollten die Mengenverhältnisse so gewählt werden, dass einerseits genügend C-Quelle und/oder Testreagenzien für eine Säurebildung durch die kariogenen Bakterien vorliegen, anderseits der Filter aber nicht verstopft wird. Die Freisetzung der C-Quelle und/oder der Testreagenzien auf dem Filter sollte erst nach 1-2 Minuten erfolgen, sodass genügend Zeit vor der Metabolisierung der C-Quelle verbleibt, um eine Anreicherung der Bakterien durch Filtration der Speichelprobe des Patienten zu ermöglichen. In einer optimalen Ausgestaltung des erfindungsgemäßen Teststreifens erfolgt die Freisetzung der C-Quelle und/oder Testreagenzien erst etwa 3-10 Minuten, vorzugsweise etwa 5-8 Minuten nach Auftragen der Speichelprobe. So können beispielsweise 1-10 mg der pulverisierten Testreagenzien mittels geeigneter Schablonen auf einen Filter aufgebracht und mit 10-50 µl, vorzugsweise mit 20 µl, eines Lacks, wie z.B. 1 Gew.-% Hydroxypropylcellulose, auf dem Filter fixiert werden. Die Filter können in einem Trockenschrank getrocknet und bis zur Verwendung bei Raumtemperatur trocken gelagert werden.

Andere für die Fixierung geeignete Lacke umfassen Polysaccharide, wie z.B. Xanthan, Chitosan, Carrageen; Polyvinylalkohole, wie z.B. Poval von Kuraray; Polyvinylakohol-PEG-Copolymere, wie z.B. Kollicoat IR von BASF; Polyvinylpyrrolidon, wie z.B. Plasdone von ISP; Vinylpyrrolidon-VinylacetatCopolymere, wie z.B. Luviskol VA von BASF; Alkylester von Poly(methylvinylether)maleinsäureanhydrid bzw. Maleinsäure-Copolymeren, wie z.B. Gantrez von ISP; Polyvinlycaprolactame, wie z.B. Luviskol Plus von BASF; Acrylat-Copolymere, wie z.B. Eudragit von Evonik oder Luvimer 100P von BASF; Poly(vinylacetat-co-crotonsäure), wie z.B. Luviset von BASF oder Vinnapas von Wacker; Vinylcaprolactam-Vinylpyrrolidondimethylaminoalkylmethacrylat-Copolymere, wie z.B. Advantage oder Aquaflex von ISP; und Polymere mit quartären (quaternären) Ammoniumverbindungen. Andere geeignete Polymere für die Fixierung der Reagenzien auf Teststreifen umfassen Celluloseether, wie z.B. Hydroxypropylcellulose, Hydroxyethylcellulose, Ethylhydroxyethylcellulose, Hydroxypropylmethylcellulose und Methylhydroxyethylcellulose. Es können darüber hinaus beliebige Kombinationen der obigen Verbindungen verwendet werden. Nachdem die in der Speichelprobe enthaltenen Mikroorganismen mit der ausgewählten C-Quelle in Kontakt gebracht worden sind, werden diese gemeinsam mit der C-Quelle unter Bedingungen inkubiert, die eine im Wesentlichen selektive Säurebildung durch die kariogenen Bakterien ermöglicht. Hierbei werden für die kariogenen Bakterien geeignete Bedingungen von Temperatur, pH, und Osmolarität gewählt, bei denen eine besonders schnelle Metabolisierung der C-Quelle durch die Bakterien erfolgen kann. Sofern mit Puffern gearbeitet wird, können diese so gewählt werden, dass sie einen pH-Wert zwischen 6,5 und 9, vorzugsweise zwischen 6,5 und 8,5 bereitstellen, wobei ein pH-Wert zwischen 7,0 und 8,5 besonders bevorzugt ist, z.B. 7,5. Wenn die Säurebildung in Gegenwart eines pH-Farbindikators erfolgt, wird der pH-Wert durch Verwendung geeigneter Puffer so eingestellt, dass zu Beginn der Inkubation der pH-Wert der Lösung oberhalb des pKs-Werts des Indikators liegt, damit bei einsetzender Säurebildung ein umgehender Farbumschlag des Indikators gewährleistet ist. So wird der pH-Wert einer Lösung, in welcher der Indikator Phenolrot enthalten ist, im Bereich von etwa 8,5 liegen, da unterhalb dieses pH-Werts Farbveränderungen bis zu einem pH-Wert von etwa 5,0 nachweisbar sind (siehe Tabelle 2).

Die Temperatur der Inkubation der Mikroorganismen mit der C-Quelle kann im Allgemeinen zwischen 20°C und 60°C liegen, vorzugsweise zwischen 25°C und 50°C, stärker bevorzugt zwischen 30°C und 45°C. Am schnellsten erfolgt die Säurebildung regelmäßig in einem Temperaturbereich zwischen 35°C und 45°C.

Erfindungsgemäß ist es besonders bevorzugt, dass die C-Quelle und/oder die Testreagenzien eine Phosphatkonzentration zwischen 1 mM und 10 mM aufweisen, wobei 5 mM besonders bevorzugt werden, wenn eine hohe Testsensitivität erwünscht ist. Anhand der verwendeten Pufferkonzentration kann die Testsensitivität auch verringert werden, um auch höhere Konzentrationen von kariogenen Bakterien im Speichel differenzieren zu können. Zur Abstufung der Sensitivität kann z.B. die Konzentration des Phosphatpuffers erhöht werden auf >10 mM, >20 mM, >30 mM, >40 mM oder sogar >50 mM. Zur Pufferung können auch andere Puffer eingesetzt werden, z.B. HEPES, MOPS, TRIS, usw. Eine weitere Möglichkeit zur Einstellung der Sensitivität besteht darin, leicht basische Salze oder Polymere in der geeigneten Menge zuzugeben.

Eine erhöhte Selektivität zu Gunsten von kariogenen Bakterien, insbesondere Mutans-Streptokokken, lässt sich ferner durch Zugabe von Aminosäuren und/oder Aminosäure-haltigen Polymeren erreichen. Zahlreiche säurebildende Bakterien der Mundhöhle sind in der Lage, die Aminogruppen von Aminosäuren enzymatisch abzuspalten. Die Freisetzung der basischen Aminogruppe führt dabei zu einer teilweisen oder vollständigen Neutralisierung der Säure, die von diesen Bakterien produziert wird. Mutans-Streptokokken, wie z.B. *S. mutans* und *S*. *sobrinus,* sind hingegen nicht in der Lage, die Aminogruppen aus Aminosäuren in nennenswertem Umfang freizusetzen, da diesen Organismen die notwendigen Enzyme in der Regel fehlen. Beispielsweise verfügen weder *S*. *mutans* noch *S*. *sobrinus* über eine Arginindeiminase. Daher ist insbesondere der Zusatz von Arginin geeignet, der Säurebildung durch Bakterien, bei denen es sich nicht um Mutans-Streptokokken handelt (z.B. *S. gordonii, S. equinus, S. mitis, S. salivarius, L. casei* und *L. fermentum, S. aureus)* entgegenzuwirken.

Wird das oben dargelegte Verfahren für die semiquantitative Bestimmung von kariogenen Bakterien dahingehend modifiziert, dass die Mikroorganismen mit der C-Quelle in Gegenwart einer Aminosäure wie z.B. Arginin und/oder Aminosäure-haltigen Polymeren inkubiert werden, so wird die Säurebildung durch *S*. *mutans* und *S*. *sobrinus* nicht beeinflusst, während die Säurebildung anderer Bakterien der Mundhöhle durch Freisetzung von basischen Aminogruppen unterdrückt wird.

In einer besonders bevorzugten Ausführungsform des erfindungsgemäßen Verfahrens werden daher die Schritte (b) und/oder (c) in Gegenwart von einer oder mehreren Aminosäure(n) und/oder einem oder mehreren Aminosäure-haltigen Polymer(en) durchgeführt. Vorzugsweise werden die Aminosäuren dem Reaktionsansatz als freie Aminosäuren zugegeben. Besonders geeignete, freie Aminosäuren, die für eine Unterdrückung der Säurebildung bei Nicht-Mutans-Streptokokken sorgen, umfassen Arginin, Asparagin, Glutamin, Lysin und Serin. Die Verwendung von Arginin als freie Aminosäure ist besonders bevorzugt. Alternativ können dem Reaktionsansatz auch ein oder mehrere Aminosäure-haltige Polymer(e) zugesetzt werden. Aminosäure-haltige Polymere umfassen kurze Peptide (z.B. Dipeptide, Tripeptide, usw.) und Polypeptide (mit einer Länge von z.B. >10, >20, >30, >40, >50 oder mehr Aminosäuren). Es können auch Mischungen von freien Aminosäuren mit Aminosäure-haltigen Polymeren verwendet werden. Eine geeignete Mischung von freien Aminosäuren und Aminosäure-haltigen Polymeren, die für eine wirksame Unterdrückung der Säurebildung bei Nicht-Mutans-Streptokokken sorgt, ist beispielsweise Pepton.

In einer weiteren besonders bevorzugten Ausführungsform wird in dem oben dargelegten Verfahren Glucose als C-Quelle verwendet und die Schritte (b) und/oder (c) werden in Gegenwart von einer Aminosäure durchgeführt, die ausgewählt ist aus der Gruppe bestehend aus Arginin, Asparagin, Glutamin, Lysin und Serin, wobei die Zugabe von Arginin besonders bevorzugt wird.

Die Aminosäure kann dem oben dargelegten Verfahren in Schritt (b) oder (c) zugesetzt werden. Die Aminosäure kann dem Reaktionsansatz, der aus der Speichelprobe, der C-Quelle und weiteren optionalen Bestandteilen besteht, in einer Menge von 0,01 bis 5 Gew.-% zugesetzt werden, z.B. in einer Menge von 0,05%, 0,1%, 0,2%, 0,3%, 0,5%, 1%, 1,5%, 2%, 2,5%, 3%, 3,5%, 4%, 4,5%, oder mehr. Eine Menge von 0,1 - 0,2 Gew.-% ist besonders bevorzugt. Sofern Aminosäure-haltige Polymere verwendet werden, kann der Fachmann geeignete Mengen der jeweiligen Polymere an Hand der obigen Angaben problemlos ermitteln.

Eine Verbesserung der Selektivität zu Gunsten von kariogenen Bakterien lässt sich außerdem auch durch die Zugabe von Harnstoff erreichen. Die Zugabe von Harnstoff sorgt insbesondere für eine Unterdrückung der Säurebildung durch *S*. *aureus.* In den oben beschriebenen Verfahren können die Schritte (b) oder (c) daher auch in der Gegenwart von Harnstoff durchgeführt werden. In einer besonders bevorzugten Ausführungsform wird in dem oben dargelegten Verfahren Saccharose als C-Quelle verwendet und die Schritte (b) oder (c) werden in Gegenwart von Harnstoff durchgeführt.

Eine weitere Verbesserung der selektiven Säurebildung kann durch Zugabe von Antibiotika erzielt werden, die in der Lage sind, das Wachstum und/oder die metabolische Aktivität von Bakterien, die im Rahmen des erfindungsgemäßen Verfahrens nicht quantifiziert werden sollen, zu hemmen und/oder zu unterbinden, wobei das Wachstum und/oder die Säurebildung der kariogenen Bakterien hingegen nicht wesentlich beeinträchtigt wird. So lässt sich beispielsweise durch Zugabe von Vancomycin, Kanamycin oder Neomycin zu der C-Quelle und/oder den Testreagenzien eine besonders hohe Spezifität für Lactobacillen erreichen, da diese sich durch die genannten Antibiotika hinsichtlich ihres Wachstums oder ihrer metabolischen Aktivität kaum hemmen lassen, während z.B. Streptokokken und Staphylokokken deutlich gehemmt werden. In ähnlicher Weise lassen sich durch Verwendung von Bacitracin und Metronidazol Bedingungen schaffen, die besonders für die selektive Säurebildung durch Mutans-Streptokokken geeignet sind. Geeignete Antibiotika können der C-Quelle und/oder den Testreagenzien in einer Menge von 0,01 Gew.-% bis 2 Gew.-% zugegeben werden, vorzugsweise in einer Menge von 0,1 bis 1 Gew.-%, z.B. 0,2 Gew.-%. Besonders geeignete Antibiotika, die das Wachstum und/oder die metabolische Aktivität von Bakterien der Gattung Lactobacillus hemmen, sind beispielsweise Vancomycin, Clindamycin und Nisin.

Tabelle 1 zeigt die Auswirkungen der Zugabe von Antibiotika auf die zeitliche Entwicklung der Säurebildung von verschiedenen kariogenen und nicht-kariogenen Bakterien, die mit 1% Glucose in Gegenwart des Indikators Bromkresolpurpur inkubiert wurden. Die genaue Zusammensetzung der Testlösungen ist in Tabelle 3 beschrieben.

**Tabelle 1: Zeiten, bis Phenolrot vollstandig auf gelb umgeschlagen ist als Funktion des Antibiotikums, das einer Glucose-Testlosung beigegeben wurde.**

| *Nr*. | *Chemikalien zusatz* | *Konz*% | *S*. *mutans* | *S. sobrinus* | *L. casei* | *L*. *rhamnosus* | *S*. *mitis* | *S*. *aureus* |
|---|---|---|---|---|---|---|---|---|
| 87 | keiner (Glucose 1%) | | 4 h | 2 h | 1 h | 1 h | 2 h | 4 h |
| 117 | Vancomycin | 0.5 | > 24 h | > 24 h | 2 h | 2 h | > 24 h | 24 h |
| 119 | Clindamycin | 0.2 | > 24 h | > 24 h | 2 h | 2 h | 2 h | > 24 h |
| 122 | Chloramphenicol | 0.2 | > 24 h | 2 h | 1 h | 1 h | 2 h | 4 h |
| 124 | Clotrimazol | 0.2 | > 24 h | 2 h | 1 h | 1 h | 1 h | 4 h |
| 126 | Metronidazol | 0.2 | 4 h | 2 h | 1 h | 1 h | 1 h | 4 h |
| 127 | Streptomycm | 0.2 | > 24 h | 4 h | 2 h | 2 h | 2 h | 24 h |
| 129 | Neomycin | 0.2 | > 24 h | > 24 h | 2 h | 4 h | > 24 h | 24 h |
| 130 | Kanamycinsulfat | 0.2 | > 24 h | > 24 h | 2 h | 4 h | > 24 h | 24 h |
| 131 | Gentamicinsulfat | 0.2 | > 24 h | > 24 h | 2 h | 4 h | > 24 h | 24 h |
| 143 | Nisin | 0.1 | 24 h | 24 h | 1 h | 1 h | > 24 | > 24 |

Die Inkubationszeit, die für die Inkubation der Mikroorganismen mit der erfindungsgemäß gewählten C-Quelle ausgewählt wird, richtet sich nach den zu quantifizierenden kariogenen Bakterien sowie nach der Art und Konzentration der C-Quelle. Die Inkubationszeit wird im Allgemeinen so gewählt werden, dass die möglichst starke Säurebildung durch die zu quantifizierenden kariogenen Bakterien erreicht wird, während eine Säurebildung durch nicht-kariogene Bakterien weitestgehend ausgeschlossen sein sollte.

Nach Inkubation mit der C-Quelle wird die Säurebildung durch die kariogenen Bakterien verfolgt, indem man mindestens einmal innerhalb eines Zeitraums von 12 Stunden nach Zugabe der C-Quelle den pH-Wert bestimmt. Der Zeitraum, in dem die Messungen erfolgen, erstreckt sich auf maximal 12 Stunden nach Zugabe der C-Quelle, es ist allerdings bevorzugt, dass der Zeitraum weniger als 10, 8, 6, 5, 4, 3, 2 oder sogar weniger als eine Stunde beträgt. Besondere Vorteile des Verfahrens ergeben sich, wenn der Zeitraum, in dem die Messung des pH-Werts erfolgt und der somit den zeitlichen Rahmen des Verfahrens bildet, 0,25 bis 3 Stunden umfasst. Dies ermöglicht die Bestimmung der kariogenen Bakterien und das damit verbundene Kariesrisiko im Rahmen einer zahnärztlichen Untersuchung. Es ist ferner bevorzugt, dass in dem genannten Zeitraum mehr als eine pH-Messung erfolgt. Vorzugsweise werden in dem Zeitraum 2, 3, 4, 5, 6, 7, 8, 9, 10 oder mehr Messungen vorgenommen. So können z.B. Messungen in regelmäßigen Intervallen von 5, 10 oder 15 Minuten über einen Zeitraum von 1, 2, 3 oder 4 Stunden erfolgen.

Eine Verbesserung bezüglich der Geschwindigkeit der messbaren Säurebildung ergibt sich bei Durchführung des erfindungsgemäßen Verfahrens in Gegenwart von Inhibitoren des Enzyms Carbonsäure-Anhydrase, wie z.B. Acetazolamid, Methazolamid, Dorzolamid und Topiramate.

Bei Entnahme einer Speichelprobe aus einem zu untersuchenden Individuum kommt es zunächst zu einem Entweichen von Kohlendioxid aus der Probe. Speichel enthält Hydrogencarbonat, das durch Aufnahme eines Protons zu Kohlendioxid und Wasser reagiert. Diese Reaktion wird durch das Enzym Carbonsäure-Anhydrase katalysiert. Durch das Entweichen von Kohlendioxid steigt der pH-Wert der Speichelprobe unmittelbar nach der Entnahme von etwa 7,4 auf 8,0 oder mehr an. Bei Zugabe einer entsprechenden C-Quelle zu der Probe und Einsetzen der Säurebildung muss zunächst dieser anfängliche pH-Anstieg kompensiert werden, was die Reaktionsdauer unnötig verlängert. Die Zugabe von Inhibitoren des Enzyms Carbonsäure-Anhydrase, wie z.B. Acetazolamid, Methazolamid, Dorzolamid und Topiramate, zu Schritt (b) oder (c) des erfindungsgemäßen Verfahrens kann daher die Dauer des gesamten Verfahrens verkürzen.

Der Inhibitor kann dem aus den Mikroorganismen der Speichelprobe, der C-Quelle und den weiteren optionalen Bestandteilen bestehenden Reaktionsansatz in einer Menge von 0,01 bis 5 Gew.-% zugesetzt werden, z.B. in einer Menge von 0,05%, 0,1%, 0,2%, 0,3%, 0,5%, 1%, 1,5%, 2%, 2,5%, 3%, 3,5%, 4%, 4,5%, oder mehr. Eine Menge von 0,1 Gew.-% ist besonders bevorzugt.

Die Bestimmung des pH-Werts kann in dem oben genannten Zeitraum mittels einer pH-Elektrode erfolgen. Die Verwendung einer pH-Elektrode setzt voraus, dass die Inkubation der Mikroorganismen aus der Plaque- oder Speichelprobe mit der C-Quelle in einer wässrigen Lösung erfolgt. Da die Abnahmerate, d.h. die auf eine Zeiteinheit bezogene Abnahme des pH-Werts, unter den oben beschriebenen Bedingungen mit der Konzentration der kariogenen Bakterien in der Probe korreliert, kann eine quantitative oder semiquantitative Bestimmung der kariogenen Bakterien in der Ausgangsprobe durch Vergleich des gemessenen pH-Werts mit einem Referenzwert, der zuvor auf Basis einer Probe mit einer bekannten Anzahl der jeweiligen kariogenen Bakterien zum selben Zeitpunkt der Reaktion und unter denselben Bedingungen erhalten wurde, erfolgen. Vorzugsweise wird mehr als eine Messung vorgenommen, wobei für jeden Zeitpunkt, zu dem eine Messung erfolgt, mindestens ein entsprechender Referenzwert vorliegt. Beispielsweise können pH-Messungen in einem Zeitraum von insgesamt 4 Stunden nach 1, 2, 3 und 4 Stunden erfolgen (siehe Beispiele). Üblicherweise werden für jeden der definierten Zeitpunkte, zu denen die Messung des pH-Werts erfolgt, mehrere Referenzwerte vorliegen, die unter Verwendung von bekannten Konzentrationen der entsprechenden kariogenen Bakterien erhalten wurden. Eine hohe Anzahl von Referenzwerten, die jeweils den pH-Wert zu einem definierten Zeitpunkt nach Zugabe der C-Quelle für eine bestimmte Konzentration der kariogenen Bakterien widerspiegeln, ermöglicht eine verhältnismäßig genaue quantitative oder semiquantitative Bestimmung von kariogenen Bakterien in einer Probe unbekannter Konzentration. So können die Referenzwerte bei der Bestimmung von Mutans-Streptokokken z.B. Konzentrationen von 10³ cfu, 10⁴ cfu, 10⁵ cfu und 10⁶ cfu entsprechen.

In einer alternativen Ausführungsform erfolgt die Bestimmung des pH-Werts mit Hilfe eines pH-Indikators, der bei Säurebildung die Farbe ändert. Der pH-Indikator wird vorzugsweise gemeinsam mit der C-Quelle zu den kariogenen Bakterien gegeben, z.B. durch gemeinsame Immobilisierung von pH-Indikator und C-Quelle auf einem Teststreifen oder durch gleichzeitige Zugabe von pH-Indikator und C-Quelle (vorzugsweise in Form einer einzigen Lösung) zu einer verdünnten, unverdünnten oder aufkonzentrierten Plaque- oder Speichelprobe. Verschiedene pH-Indikatoren haben sich als nützlich für die Durchführung des erfindungsgemäßen Verfahrens erwiesen. Diese umfassen z.B. die Indikatoren Bromkresolpurpur, Bromkresolgrün, m-Kresolpurpur, Bromthymolblau, Phenolrot und Chlorphenolrot. Tabelle 2 zeigt die Farbveränderung dieser Indikatoren als Funktion des pH-Werts. Die Inkubationszeit, die angelegt werden muss, bis eine Veränderung der Farbe nachgewiesen werden kann, hängt von den verwendeten C-Quellen und Testreagenzien sowie deren absoluten und relativen Mengenverhältnissen zueinander ab. In einer bevorzugten Ausführungsform beträgt der Zeitraum bis zur Veränderung der Farbe des pH-Indikators weniger als 60 Minuten, weniger als 45 Minuten, weniger als 30 Minuten, weniger als 20 Minuten oder weniger als 15 Minuten nach Mischen von Bakterien, C-Quelle und Indikator.

Die Geschwindigkeit der Farbreaktion kann u.a. über die Konzentration des Puffers in Reaktionsansatz gesteuert werden. Es hat sich beispielsweise bei Lösungen, die den Indikator Phenolrot enthielten, gezeigt, dass eine Erhöhung der Konzentration des Phosphatpuffers von 5 mM auf 50 mM die Farbreaktion um etwa den Faktor 3 verlangsamte. Mittels der Pufferkonzentration kann also die Sensitivität dieses Kariesrisikotests eingestellt werden.

**Tabelle 2: pH-Indikatoren und deren Farbe als Funktion des pH-Werts.**

| | **m-Kresolpurpur** | **Phenolrot** | **Bromthymolblau** | **Bromkresolpurpur** | **Chlorophenolrot** | **Bromkresolgrün** |
|---|---|---|---|---|---|---|
| **pK** | **8.2** | **7.9** | **7** | **6** | **5.6** | **4.7** |
| 9.0-8.5 | violett | | | | | |
| 8.5-8.0 | braun | dunkelrot | Dunkelblau | | | |
| 8.0-7.5 | hellbraun | rot | petrol | | | |
| 7.5-7.0 | dunkelgelb | rot | grun-blau | violett | | |
| 7.0-6.5 | gelb | orangerot | grun | bordeaux | purpur | |
| 6.5-6.0 | | orange | grun-gelb | rotbraun | violett | |
| 6.0-5.5 | | orange-gelb | gelb | grunviolett | rot | dunkelblau |
| 5.5-5.0 | | gelb | | dunkelgelb | orange | petrol |
| 5.0-4.5 | | | | gelb | orange | grun-blau |
| 4.5-4.0 | | | | | gelb | grun |
| 4.0-3.5 | | | | | | grun-gelb |
| 3.5-3.0 | | | | | | gelb |

Die durch den Indikator verursachte Farbänderung wird mit einem Farbstandard verglichen, der zuvor unter gleichen Bedingungen mit Proben erhalten wurde, die eine bekannte Anzahl der jeweiligen kariogenen Bakterien enthielten. Dieser Vergleich mit dem Farbstandard ermöglicht die semiquantitative Bestimmung der kariogenen Bakterien in der ursprünglichen Probe. Besonders bevorzugt ist es wiederum, dass der Farbstandard mehrstufig ist, d.h. er umfasst für jeden der definierten Zeitpunkte, zu denen die Messung des pH-Werts erfolgt, mehrere Farbtiefen, die sich einer bestimmten Konzentration der kariogenen Bakterien in der ursprünglichen Speichelprobe zuordnen lassen. Vorzugsweise handelt es sich bei dem Farbstandard um einen solchen, bei dem sich die Unterschiede hinsichtlich der Konzentration mit bloßem Auge, d.h. ohne technische Vorrichtungen, erkennen lassen. Bei den einzelnen Stufen des Standards kann es sich z.B. um Zehnerlogarithmen der Konzentration der Bakterien, wobei die Konzentration in Kolonie-bildenden Einheiten (cfu) gemessen wird. So kann z.B. der Farbstandard, mit dessen Hilfe sich die Konzentration der Mutans-Streptokokken abschätzen lässt, vier Stufen umfassen, die Konzentrationen von 10³ cfu, 10⁴ cfu, 10⁵ cfu und 10⁶ cfu entsprechen. Die bei dem erfindungsgemäßen Testverfahren erhaltene Farbveränderung kann mit den vier vorgegebenen Farbintensitäten verglichen werden. In der Regel wird sich damit eine Aussage über die Konzentration der kariogenen Bakterien in der untersuchten Plaque- oder Speichelprobe treffen lassen; z.B. kann festgestellt werden, dass die im Rahmen des Tests erhaltene Farbintensität auf eine Konzentration von Mutans-Streptokokken im Bereich von zwischen 10⁴ und 10⁵ cfu schließen lässt. Dies verweist auf ein hohes Kariesrisiko.

In einem weiteren Aspekt betrifft die Erfindung auch einen Teststreifen zur Durchführung eines wie oben beschriebenen Verfahrens, wobei auf der Oberfläche des Testreifens mindestens eine C-Quelle und ein pH-Indikator derart immobilisiert wurden, dass sie bei Inkontaktbringen des Testreifens mit einer Bakteriensuspension vom Testreifen freigesetzt werden in sich in der flüssigen Bakteriensuspension lösen. Die C-Quelle wird wie oben beschrieben so ausgewählt, dass sie selektiv von kariogenen Bakterien, wie z.B. Mutans-Streptokokken oder Lactobacillen, zu Säure fermentiert wird. Der pH-Indikator ist in der Lage, bei Säurebildung die Farbe zu ändern, wobei die Änderung vorzugsweise mit bloßem Auge wahrnehmbar ist. Es ist ausreichend, dass der Teststreifen eine der beiden genannten Komponenten in immobilisierter Form umfasst. Die jeweils andere Komponente kann dem Teststreifen zugesetzt werden, z.B. durch Auftropfen. In einer bevorzugten Ausführungsform sind sowohl die C-Quelle als auch der Indikator auf der Oberfläche des Teststreifens immobilisiert. Die C-Quelle und/oder der pH-Indikator können mittels eines Lacks (siehe oben) auf dem Teststreifen immobilisiert werden.

Der Teststreifen umfasst vorzugsweise ferner ein Filtermaterial (oder besteht aus diesem) mit einer Porengröße im Bereich von 1-10 µm. Bevorzugte Porengrößen liegen im Bereich von 2-8 µm, z.B. 2, 3, 4, 5, 6, 7 oder 8 µm. Auf diesem Filtermaterial ist die C-Quelle und/oder der pH-Indikator immobilisiert. Dies hat den Vorteil, dass das Filtermaterial unmittelbar zur Anreicherung der kariogenen Bakterien verwendet werden kann.

Die Erfindung betrifft auch einen Testkit zur Durchführung eines wie oben beschriebenen Verfahrens, umfassend:
(a) eine C-Quelle, die von Gruppe von kariogenen Bakterien zu Säure fermentiert wird, wobei die C-Quelle ausgewählt ist aus der Gruppe bestehend aus Maltotriose, Mannose und N-Acetylglucosamin und wobei die kariogenen Bakterien ausgewählt sind aus der Gruppe bestehend aus Mutans-Streptokokken und Lactobacillen;
(b) einen pH-Indikator, der bei Säurebildung die Farbe ändert;
(c) einen Farbstandard, der die semiquantitative Bestimmung der Konzentration der kariogenen Bakterien in der Probe ermöglicht.

Vorzugsweise handelt es sich bei dem Farbstandard um einen wie oben beschriebenen mehrstufigen Standard, der als Referenz dient. Der Kit kann darüber hinaus ferner Instruktionen zur Durchführung des Verfahrens umfassen. Der Kit kann selbstverständlich auch einen oder mehrere der wie oben beschriebenen Testreifen umfassen.

### BESCHREIBUNG DER FIGUREN

Figur 1 zeigt die Kinetik der Säurebildung von Reinkulturen. Die Buchstaben A-G bezeichnen den Zeitpunkt, zu dem erstmalig anhand eines pH-Indikators eine Säureproduktion nachgewiesen wurde. Die Zahlen 0-5 geben die Stufe der pH-Reduktion nach 1, 2 und 4 h an. Es gilt somit die folgende Kodierung: <Zeitpunkt des Farbumschlags> <Farbstufe nach 1h> <Farbstufe nach 2h> <Farbstufe nach 4h>.

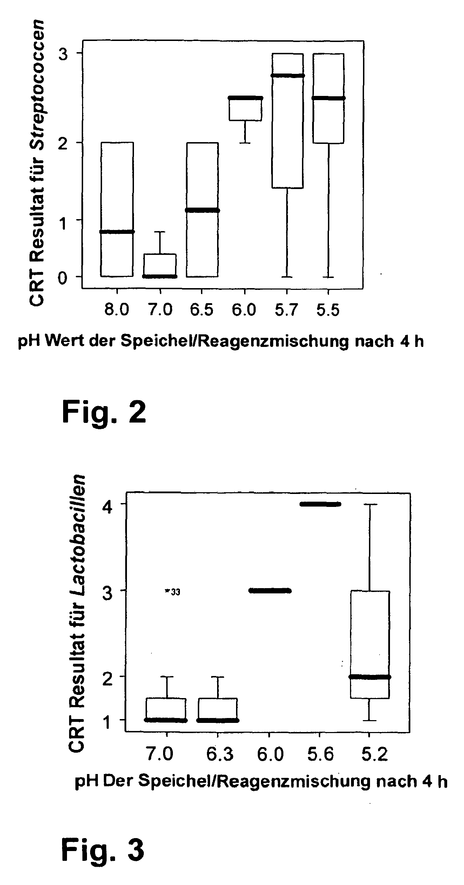

Figur 2 zeigt die Korrelation zwischen den Ergebnissen von CRT bacteria und dem erfindungsgemäßen Verfahren unter Verwendung von Testlösung Nr. 136 nach 4 h für Mutans-Streptokokken. Es ist ersichtlich, dass die Risikoschwelle von >10⁵ CFU/ml einer pH-Reduktion auf weniger als etwa 6,5 entspricht.

Figur 3 zeigt die Korrelation zwischen den Ergebnissen von CRT bacteria und dem erfindungsgemäßen Verfahren unter Verwendung von Testlösung Nr. 98 nach 4 h für Lactobacillen. Es ist ersichtlich, dass die Risikoschwelle von >10⁵ CFU/ml einer pH-Reduktion auf weniger als etwa 6,0 entspricht.

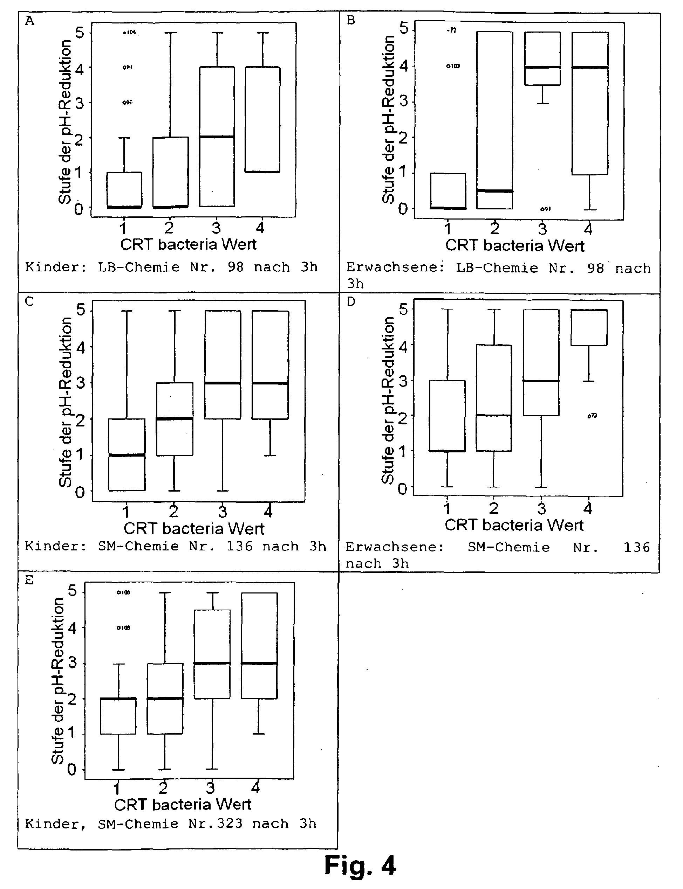

Figur 4 zeigt die Korrelation zwischen den Ergebnissen von CRT bacteria und dem erfindungsgemäßem Verfahren unter Verwendung von (A, B) Testlösung Nr. 98, (C, D) Testlösung Nr. 136 oder (E) Testlösung Nr. 323 bei (A, C, E) Kindern und (B, C) Erwachsenen nach 3 Stunden für (A, B) Lactobacillen und (C, D) Mutans-Streptokokken. Die Zahlen 0-5 auf der Y-Achse geben die Stufe der pH-Reduktion an (vergleiche Figur 1). Es ist ersichtlich, dass mit zunehmendem CRT bacteria Wert (X-Achse) auch die Stufen der pH Reduktion zunehmen.

### BEISPIELE

### Bakterienstämme

Stockkulturen der für die Versuche ausgewählten Stämme wurden auf CRYOBANK (Mast Diagnostica, CRYO/M) Keramikträgern bei einer Temperatur von -80°C gelagert. Zur Anzucht wurde ein Keramikringchen in 10 ml Nährmedium gegeben und 14-72 h inkubiert. Diese Kultur wurde als Passage 0 (P0) bezeichnet. Anschließend wurden 100 µl Kultur in 10 ml frisches Medium überimpft und für 24 h inkubiert. Kulturen der Passage 2 wurden für die nachstehenden Experimente zur Säureproduktion verwendet. Die Kulturen wurden dabei soweit verdünnt, bis eine Absorption bei 650 nm von etwa 0.6-1.0 erhalten wurde. Die Bakteriensuspensionen wurden bei 8000 UpM für 5 Minuten zentrifugiert. Der Kulturüberstand wurde entfernt, und das Pellet wurde im selben Volumen Wasser (10 ml, deionisiert, steril) resuspendiert. Die Probe wurde erneut zentrifugiert (8000 UpM, 5 Minuten), und der Überstand wurde entfernt. Das Pellet wurde anschließend in 1 ml deionisiertem Wasser resuspendiert, was 1/10 des Ursprungsvolumen entspricht.

### Säureproduktion in vitro

Zur Messung der Säureproduktion wurden 40 µl Bakteriensuspension in 96-Well Polystyrol-Mikrotiterplatten oder in 1,5 ml Mikrozentrifugenröhrchen zu 100 µl einer Testlösung gegeben, die neben einem geeigneten pH-Indikator (z.B. Phenolrot und Bromkresolpurpur) eine fermentierbare C-Quelle, 5 mM Phosphat oder 5 mM Tris-HCl und ggf. ein Antibiotikum oder eine Aminosäure, wie z.B. Arginin, enthielt. Die verwendeten Testlösungen wurden auf einen definierten Ausgangs-pH-Wert eingestellt. Bei der Herstellung der Testlösungen wurden die Komponenten in 75% des gewünschten Endvolumens an deionisiertem Wasser gelöst. Dann wurde der entsprechende pH-Wert eingestellt, und es wurde mit deionisiertem Wasser auf das berechnete Volumen aufgefüllt. Die hergestellten Lösungen wurden im Kühlschrank gelagert und nicht länger als 2 Wochen benutzt.

Nach Zugabe der Bakteriensuspension zu der jeweiligen Testlösung wurde in regelmäßigen Abständen der Farbumschlag des pH-Indikators visuell gegen eine Referenzprobe (nur Testlösung) beurteilt. Dazwischen wurden die Proben bei 37°C im Inkubator gelagert. Die Säurebildung wurde durch die Farbveränderungen der Testlösungen visuell (ohne Unterstützung durch optische Messgeräte) beschrieben. Die Ergebnisse der Untersuchungen sind in der in Figur 1 dargestellten Tabelle aufgeführt.

Die Zusammensetzung der im Rahmen der vorliegenden Erfindung verwendeten Testlösungen ist in der nachstehenden Tabelle 3 beschrieben.

**Tabelle 3: Zusammensetzungen der Testlösungen.**

| **Nr.** | **Indikator** | **Substrat** | **Konz (%)** | **Zusätze** | **Puffer** | **pH** |
|---|---|---|---|---|---|---|
| 87 | Phenolrot | Glucose | 1 | | Phosphat, 5 mM | 8.5 |
| 98 | Bromkresolpurpur | Lactose | 1 | | Phosphat, 5 mM | 7.3 |
| 117 | Phenolrot | Glucose | 1 | Vancomycin, 0,5 % | Phosphat, 5 mM | 8.5 |
| 119 | Phenolrot | Glucose | 1 | Clindamycin, 0,2% | Phosphat, 5 mM | 8.5 |
| 122 | Phenolrot | Glucose | 1 | Chloramphenicol, 0,2% | Phosphat, 5 mM | 8.5 |
| 124 | Phenolrot | Glucose | 1 | Clotrimazol, 0,2% | Phosphat, 5 mM | 8.5 |
| 126 | Phenolrot | Glucose | 1 | Metronidazol, 0,2% | Phosphat, 5 mM | 8.5 |
| 127 | Phenolrot | Glucose | 1 | Streptomycin, 0,2% | Phosphat, 5 mM | 8.5 |
| 129 | Phenolrot | Glucose | 1 | Neomycin, 0,2% | Phosphat, 5 mM | 8.5 |
| 130 | Phenolrot | Glucose | 1 | Kanamycinsulfat, 0,2% | Phosphat, 5 mM | 8.5 |
| 131 | Phenolrot | Glucose | 1 | Gentamycinsulfat, 0,2% | Phosphat, 5 mM | 8.5 |
| 136 | Phenolrot | Saccharose | 20 | | Phosphat, 5 mM | 8.5 |
| 143 | Phenolrot | Glucose | 1 | Nisin, 0.1% | Phosphat, 5 mM | 8.5 |
| 209 | Phenolrot | Saccharose | 20 | | Phosphat, 50 mM | 8.5 |
| 237 | Phenolrot | Saccharose | 20 | Arginin 0.1% | Phosphat, 5 mM | 8.5 |
| 323 | Phenolrot | Maltose | 20 | | Phosphat, 5 mM | 8.5 |
| 337 | Phenolrot | Saccharose | 20 | Harnstoff 0.1% | Phosphat, 5 mM | 8.5 |
| 344 | Phenolrot | Maltose | 20 | Arginin 0.1% | Phosphat, 5 mM | 8.5 |
| 354 | Phenolrot | Saccharose | 20 | Aspargin 0.1% | Phosphat, 5 mM | 8.5 |
| 355 | Phenolrot | Saccharose | 20 | Lysin 0.1% | Phosphat, 5 mM | 8.5 |
| 358 | Phenolrot | Saccharose | 20 | Serin 0.1% | Phosphat, 5 mM | 8.5 |
| 408 | Phenolrot | Saccharose | 20 | Glutamin 0.1% | Phosphat, 5 mM | 8.5 |
| 409 | Phenolrot | Glucose | 1 | Arginin 0.1% | Phosphat, 5 mM | 8.5 |
| 410 | Phenolrot | Glucose | 1 | Aspargin 0.1% | Phosphat, 5 mM | 8.5 |
| 411 | Phenolrot | Glucose | 1 | Glutamin 0.1% | Phosphat, 5 mM | 8.5 |
| 412 | Phenolrot | Glucose | 1 | Serin 0.1% | Phosphat, 5 mM | 8.5 |
| 413 | Phenolrot | Glucose | 1 | Harnstoff 0.1% | Phosphat, 5 mM | 8.5 |

Zusätzlich wurde wie oben beschrieben überprüft, welchen Einfluss die Zugabe unterschiedlicher Aminosäuren oder Harnstoff auf die Zeitspanne hat, in der der Indikator Phenolrot vollständig auf gelb umschlägt (siehe folgende Tabelle 4).

**Tabelle 4: Zeiten, bis Phenolrot vollständig auf gelb umgeschlagen ist, wenn den Kohlenstoffquellen Aminosäuren zugegeben wurden. kD: keine Daten.**

| *Nr*. | *Kohlenstoff* | *Zusatz* | *S*. *mutans* | *S. sobrinus* | *S. gordonii* | *L*. *fermentum* | *S. aureus* |
|---|---|---|---|---|---|---|---|
| 136 | Saccharose 20% | | 2 h | 1 h | 2 h | 3 h | 4 h |
| 355 | Saccharose 20% | Lysin 0.1% | 2 h | 4 h | 24 h | kD | 24 h |
| 237 | Saccharose 20% | Arginin 0.1% | 2 h | 2 h | 24 h | > 24 h | > 24 h |
| 354 | Saccharose 20% | Aspargin 0.1% | 2 h | 4 h | > 24 h | > 24 h | > 24 h |
| 408 | Saccharose 20% | Glutamin 0.1% | 2 h | 4 h | > 24 h | > 24 h | 24 h |
| 358 | Saccharose 20% | Serin 0.1% | 2 h | 2 h | 4 h | > 24 h | 24 h |
| 337 | Saccharose 20% | Harnstoff 0.1% | 1 h | 1 h | 4 h | 2 h | 24 h |
| 87 | Glucose 1% | | 1 h | 1 h | 2 h | 3 h | 2 h |
| 409 | Glucose 1% | Arginin 0.1% | 1 h | 1 h | > 24 h | 24 h | 4 h |
| 410 | Glucose 1% | Aspargin 0.1% | 2 h | 1 h | 4 h | > 24 h | 4 h |
| 411 | Glucose 1% | Glutamin 0.1% | 2 h | 1 h | 4 h | 24 h | 4 h |
| 412 | Glucose 1% | Serin 0.1% | 2 h | 1 h | 4 h | 24 h | 4 h |
| 413 | Glucose 1% | Harnstoff 0.1% | 1 h | 1 h | 4 h | 2 h | 2 h |

### Klinische Untersuchungen mit Speichelproben

Um zu überprüfen, ob die semiquantitative Bestimmung von kariogenen Bakterien in einer Speichelprobe gemäß dem Verfahren der vorliegenden Erfindung mit den Ergebnissen des bewährten Kariesrisikotests CRT^{©} bacteria korreliert, wurden ca. 80 Speichelproben parallel mit CRT^{©} bacteria Test und den Testlösungen 136 und 98 der vorliegenden Erfindung (siehe Tabelle 3) untersucht.

Die Versuchspersonen wurden angewiesen, 30 Sekunden auf einem Paraffinpellet (CRT, Ivoclar Vivadent AG, Schaan, Liechtenstein) zu kauen und den stimulierten Speichel auszuspucken. Anschließend wurden die Personen angewiesen, weitere 5 Minuten auf dem Paraffinpellet zu kauen, und der Speichel wurde in einem 50 ml-Kunstoffröhrchen mit Schaubdeckel gesammelt. Der CRT^{©}bacteria Test erlaubt den parallelen, semiquantitativen Nachweis von im Speichel vorhandenen Mutans-Streptokokken und Lactobacillen und eine Einteilung des Patienten in eine von vier verschiedenen Risikostufen. Der Test wurde wie folgt durchgeführt.
1. Den Agarträger aus dem Probenröhrchen entnehmen.
2. Eine NaHCO₃-Tablette auf den Boden des Röhrchens geben.
3. Schutzfolien von beiden Agarflächen vorsichtig abziehen; Agarflächen dabei nicht berühren.
4. Jede Agarfläche mit Hilfe einer Pipette mit 50 µl Speichel vollständig benetzen, ohne die Agarfläche zu zerkratzen.
5. Überschüssigen Speichel abtropfen lassen.
6. Den Agarträger in das Röhrchen zurückgeben und fest verschließen.
7. Das Röhrchen 48-72 Stunden bei 37°C im Brutschrank, z.B.
   Cultura/Ivoclar Vivadent, aufrechtstehend aufbewahren.
8. Nach Entnahme des Röhrchens aus dem Brutschrank die Koloniendichte der Mutans-Streptokokken bzw. Lactobacillen jeweils mit den entsprechenden Abbildungen der beiliegenden Standards vergleichen.

Es wurde eine Einteilung in die Stufen CRT 0-1 (entspricht etwa 10³ cfu), CRT 1-2 (entspricht etwa 10⁴ cfu), CRT 2-3 (entspricht etwa 10⁵ cfu) und CRT 3 (entspricht etwa 10⁵ cfu) vorgenommen. Aliquots von jeweils 50 µl derselben Speichelproben wurden anschließend mit 50 µl der Testlösung 136 (für Mutans-Streptokokken) bzw. Testlösung 98 (für Lactobacillen) inkubiert, und der pH-Wert wurde jeweils 1, 2, 3 und 4 Stunden nach Zugabe der C-Quelle zu den Proben mit Hilfe einer pH-Elektrode oder anhand der farblichen Veränderung des Indikators gemessen. Die Ergebnisse sind nachstehend zusammengefasst.

| | **pH-Wert mit Testlösung 136** | | | |
|---|---|---|---|---|
| | **1 h** | **2 h** | **3 h** | **4 h** |
| CRT 0-1 | 7.2 | 6.9 | 6.6 | 6.2 |
| CRT 1-2 | 7 | 6.7 | 6.3 | 6 |
| CRT 2-3 | 6.9 | 6.5 | 5.9 | 5.7 |
| CRT 3 | 6.8 | 6 | 5.5 | <5.5 |

| | **pH Wert mit Testlösung 98** | | | |
|---|---|---|---|---|
| | **1 h** | **2 h** | **3 h** | **4 h** |
| CRT 1-2 | 7.2 | 7 | 6.8 | 6.5 |
| CRT 2-3 | 7.1 | 6.8 | 6.5 | 6 |
| CRT 3-4 | 7 | 6.5 | 6 | 5.5 |
| CRT 4 | 6.8 | 6 | 5.5 | 5 |

Es konnte gezeigt werden, dass die Korrelation zwischen dem CRT bacteria und den gemessenen pH-Werten statistisch signifikant war (siehe Figuren 2 und 3).

Um zu überprüfen, ob die semiquantitative Bestimmung von kariogenen Bakterien in einer Speichelprobe gemäß dem Verfahren der vorliegenden Erfindung sowohl in Kindern als auch in Erwachsenen mit den Ergebnissen des bewährten Kariesrisikotests CRT^{©} bacteria korreliert, wurden unter Verwendung der Testlösungen 136 und 323 für Mutans-Streptokokken und der Testlösung 98 für Lactobacillen 69 Speichelproben von Erwachsenen und 111 Speichelproben von Kindern parallel mit dem CRT^{©} bacteria Test und den Testlösungen der vorliegenden Erfindung untersucht.

Der CRT^{©} bacteria Test und die entsprechende Einteilung in Stufen wurde wie oben beschrieben vorgenommen (vergleiche "Klinische Untersuchungen mit Speichelproben"). Nach Durchführung des CRT^{©} bacteria Tests wurden Aliquots derselben Speichelproben anschließend 1:1 mit der Testlösung 136 oder 323 (für Mutans-Streptokokken) bzw. Testlösung 98 (für Lactobacillen) inkubiert, und der pH-Wert wurde 3 Stunden nach Zugabe der C-Quelle zu den Proben anhand der farblichen Veränderung des Indikators gemessen. Die Ergebnisse sind in Figur 4 dargestellt. Es ist deutlich zu erkennen, dass die Stufen der Säurebildung bei Speichelproben mit Testlösung 98 mit den CRT^{©} bacteria Werten für Lactobacillen korrelieren (Figur 4A, 4B). Ebenso korrelieren die Stufen der Säurebildung bei Speichelproben, die mit Testlösung 136 (Figur 4C, 4D) und 323 (Figur 4E) verwendet wurden, mit den aus dem CRT^{©} bacteria Test erhaltenen Ergebnissen für Mutans-Streptokokken.

Die Ergebnisse der statistischen Analysen sind in folgender Tabelle 5 zusammengefasst:

**Tabelle 5: Statistische Analyse der Korrelation von CRT bacteria und verschiedenen Testlösungen nach Spearmans Rangkorrelationskoeffizient (Spearmans Rho) (N = Probengröße, p = Wahrscheinlichkeit, dass Ergebnis zufallsbasiert ist).**

| | **Erwachsene** | | | **Kinder** | | |
|---|---|---|---|---|---|---|
| | N | Korrelation | p | N | Korrelation | p |
| **SM 136, 3h** | 69 | 0,51 | < 0,001 | 110 | 0,40 | < 0,001 |
| **SM 323, 3h** | nicht getestet | | | 110 | 0.37 | < 0,001 |
| **LB 98, 3h** | 70 | 0,36 | 0,002 | 111 | 0,28 | 0,003 |

### Untersuchungen zur Aufkonzentrierung von Speichelproben

Um zu überprüfen, ob durch Aufkonzentrierung von Speichelproben mittels passiver Filtration die für einen Test benötigte Zeit verkürzt werden kann, wurde der Nachweis von Mutans-Streptokokken in 9 konzentrierten und 9 nicht-konzentrierten Speichelproben verglichen. Zu diesem Zweck wurden Löcher mit einem Durchmesser von 5 mm in ein Kunststoffplättchen gebohrt. Diese Löcher wurden von unten mit einem Filtermaterial (Glasfaser) verschlossen. Plättchen und Filter wurden so auf ein Saugvlies (Cellulose) platziert, dass die Filter zwischen dem Kunststoffplättchen und dem Saugvlies angeordnet waren. 60 µl Speichelprobe wurden durch eines der Löcher auf den Filter gegeben. Anschließend wurden 20 µl der Testlösung 136 auf den Filter gegeben. Nach Abschluss der Filtration wurde das Saugvlies von der Platte mit den Filtern getrennt, und das Kunststoffplättchen und die Filter wurden für bis zu 4 h bei 37°C inkubiert.

Zu Vergleichszwecken wurden in Mikrotiterplatten 60 µl Speichel mit 60 µl Testlösung 136 gemischt und ebenfalls für bis zu 24 h bei 37°C inkubiert. Sowohl in den Kunststoffplättchen als auch in Mikrotiterplatten wurde die Stufe der pH-Reduktion nach 30, 45, 60, 120 und 180 min Inkubation anhand der farblichen Veränderung des Indikators gemessen. Die beiden Verfahren wurden sowohl miteinander als auch mit dem CRT^{©} bacteria Test (CRT SM) verglichen (siehe Farbabstufungen von 0-5 in nachstehender Tabelle).

| | **Filterschablonen** | | **Mikrotiterplatte** | |
|---|---|---|---|---|
| **CRTSM** | **30 min** | **45 min** | **120 min** | **180 min** |
| 3 | 3 | 5 | 5 | 5 |
| 3 | 5 | 5 | 5 | 5 |
| 0 | 1 | 1 | 2 | 5 |
| 0 | 1 | 2 | 2 | 3 |
| 3 | 3 | 3 | 2 | 4 |
| 2 | 3 | 4 | 5 | 4 |
| 2 | 3 | 4 | 3 | 5 |
| 1 | 1 | 2 | 1 | 1 |
| 0 | 1 | 2 | 1 | 1 |

Die nachgewiesenen Farbumschläge korrelieren mit den gemessenen CRT Werten. Es konnte außerdem gezeigt werden, dass nach der Konzentration von Speichelproben durch einen Filter in 45 Minuten ein vergleichbarer Farbumschlag stattfindet, wie in Mikrotiterplatten in 120 min. Die oben beschriebene Konzentration der Proben erlaubt somit die Durchführung des erfindungsgemäßen Verfahrens in deutlich kürzerer Zeit.

## Patentansprüche

1. Verfahren zur semiquantitativen Bestimmung von kariogenen Bakterien in einer Plaque- oder Speichelprobe, bei dem man
(a) die in einer Plaque- oder Speichelprobe enthaltenden Mikroorganismen optional aufkonzentriert;
(b) die Mikroorganismen mit einer C-Quelle in Kontakt bringt, die von den kariogenen Bakterien zu Säure fermentiert wird;
(c) die Mikroorganismen und die C-Quelle unter Bedingungen inkubiert, die eine selektive Säurebildung durch die kariogenen Bakterien ermöglichen;
(d) mindestens einmal innerhalb eines Zeitraums von 12 Stunden nach Zugabe der C-Quelle den pH-Wert bestimmt;
wobei die semiquantitative Bestimmung der kariogenen Bakterien in der Probe durch Vergleich des in Schritt (d) bestimmten pH-Werts mit mindestens einem Referenzwert erfolgt,
wobei es sich bei den kariogenen Bakterien um Mutans-Streptokokken handelt, und wobei die C-Quelle Maltotriose ist.

2. Verfahren zur semiquantitativen Bestimmung von kariogenen Bakterien in einer Plaque- oder Speichelprobe, bei dem man
(a) die in einer Plaque- oder Speichelprobe enthaltenden Mikroorganismen optional aufkonzentriert;
(b) die Mikroorganismen mit einer C-Quelle in Kontakt bringt, die von den kariogenen Bakterien zu Säure fermentiert wird;
(c) die Mikroorganismen und die C-Quelle unter Bedingungen inkubiert, die eine selektive Säurebildung durch die kariogenen Bakterien ermöglichen;
(d) mindestens einmal innerhalb eines Zeitraums von 12 Stunden nach Zugabe der C-Quelle den pH-Wert bestimmt;
wobei die semiquantitative Bestimmung der kariogenen Bakterien in der Probe durch Vergleich des in Schritt (d) bestimmten pH-Werts mit mindestens einem Referenzwert erfolgt,
wobei es sich bei den kariogenen Bakterien um Lactobacillen handelt, und wobei die C-Quelle ausgewählt ist aus der Gruppe bestehend aus Mannose und N-Acetylglucosamin.

3. Verfahren nach einem der Ansprüche 1 oder 2, wobei der pH-Wert in Schritt (d) mit einer pH-Elektrode bestimmt wird.

4. Verfahren nach einem der Ansprüche 1 oder 2, wobei der pH-Wert in Schritt (d) mit einem pH-Indikator bestimmt wird, der bei Säurebildung seine Farbe ändert.

5. Verfahren nach Anspruch 4, wobei der pH-Indikator ausgewählt ist aus der Gruppe bestehend aus Bromkresolpurpur, Bromkresolgrün, m-Kresolpurpur, Bromthymolblau, Phenolrot und Chlorphenolrot.

6. Verfahren nach einem der vorhergehenden Ansprüche, wobei Schritt (c) in Gegenwart von einem oder mehreren Antibiotika erfolgt, die das Wachstum und/oder die Säurebildung der kariogenen Bakterien nicht wesentlich beeinträchtigt.

7. Verfahren nach einem der vorhergehenden Ansprüche, wobei die Schritte (b) und/oder (c) in Gegenwart von einer oder mehreren Aminosäure(n) und/oder einem oder mehreren Aminosäure-haltigen Polymer(en) erfolgen.

8. Verfahren nach Anspruch 7, wobei die Schritte (b) und/oder (c) in Gegenwart von einer oder mehreren Aminosäuren erfolgen, die ausgewählt sind aus der Gruppe bestehend aus Arginin, Asparagin, Glutamin, Lysin und Serin.

9. Verfahren nach Anspruch 8, wobei die Schritte (b) und/oder (c) in Gegenwart von Arginin erfolgen.

10. Teststreifen zur Durchführung eines Verfahrens nach einem der Ansprüche 4-9, wobei auf der Oberfläche des Testreifens eine C-Quelle, die von kariogenen Bakterien zu Säure fermentiert wird, und ein pH-Indikator, der bei Säurebildung die Farbe ändert, so immobilisiert wurden, dass bei Inkontaktbringen des Teststreifens mit einer Bakteriensuspension die C-Quelle und der pH-Indikator vom Testreifen freigesetzt werden, wobei die C-Quelle ausgewählt ist aus der Gruppe bestehend aus Maltotriose, Mannose und N-Acetylglucosamin und wobei die Kariogenen Bakterien ausgewählt sind aus der Gruppe bestehend aus Mutans-Streptokokken und Lactobacillen.

11. Teststreifen nach Anspruch 10, wobei der Testreifen ein Filtermaterial mit einer Porengröße von weniger als 20 µm umfasst, auf dem die C-Quelle und der pH-Indikator immobilisiert wurden.

12. Teststreifen nach Anspruch 10 oder 11, wobei die C-Quelle und/oder der pH-Indikator mittels eines Polymerlacks auf dem Teststreifen immobilisiert wurden.

13. Testkit zur Durchführung eines Verfahrens nach einem der Ansprüche 4-9, umfassend:
(a) eine C-Quelle, die von den kariogenen Bakterien zu Säure fermentiert wird, wobei die C-Quelle ausgewählt ist aus der Gruppe bestehend aus Maltotriose, Mannose und N-Acetylglucosamin und wobei die Kariogenen Bakterien ausgewählt sind aus der Gruppe bestehend aus Mutans-Streptokokken und Lactobacillen.
(b) einen pH-Indikator, der bei Säurebildung die Farbe ändert;
(c) einen Farbstandard, der die semiquantitative Bestimmung der Konzentration der kariogenen Bakterien in der Probe ermöglicht.

14. Testkit nach Anspruch 13, wobei der Farbstandard ein mehrstufiger Standard ist.

15. Testkit nach Anspruch 13 oder 14, wobei der Kit mindestens einen Testreifen nach einem der Ansprüche 10-12 umfasst.

## Claims

1. Method for the semi-quantitative determination of cariogenic bacteria in a plaque or saliva sample, comprising:
(a) optionally concentrating the microorganisms contained in the plaque or saliva sample;
(b) contacting the microorganisms with a C source which is fermented to acid by the cariogenic bacteria;
(c) incubating the microorganisms and the C source under conditions which allow for a selective formation of acid by the cariogenic bacteria;
(d) determining the pH at least once within a period of 12 hours after addition of the C source,
wherein semi-quantitative determination of the cariogenic bacteria in the sample is carried out by comparison of the pH determined in step (d) with at least one reference value,
wherein the cariogenic bacteria are mutans streptococci and wherein the C source is maltotriose.

2. Method for the semi-quantitative determination of cariogenic bacteria in a plaque or saliva sample, comprising:
(a) optionally concentrating the microorganisms contained in the plaque or saliva sample;
(b) contacting the microorganisms with a C source that is fermented to acid by the cariogenic bacteria;
(c) incubating the microorganisms and the C source under conditions which allow for a selective formation of acid by the cariogenic bacteria;
(d) determining the pH at least once within a period of 12 hours after addition of the C source,
wherein semi-quantitative determination of the cariogenic bacteria in the sample is carried out by comparison of the pH determined in step (d) with at least one reference value,
wherein the cariogenic bacteria are lactobacilli and wherein the C source is selected from the group consisting of mannose and N-acetylglucosamine.

3. Method according to any of claims 1 or 2, wherein the pH in step (d) is determined with a pH electrode.

4. Method according to any of claims 1 or 2, wherein the pH in step (d) is determined with a pH indicator which changes colour upon acid formation.

5. Method according to claim 4, wherein the pH indicator is selected from the group consisting of bromocresol purple, bromocresol green, m-cresole purple, bromothymol blue, phenol red and chlorophenol red.

6. Method according to any of the preceding claims, wherein step (c) is carried out in the presence of one or more antibiotics which do not significantly affect the growth and/or acid formation of the cariogenic bacteria.

7. Method according to any of the preceding claims, wherein steps (b) and/or (c) is/are carried out in the presence of one or more amino acids and/or one or more amino acid containing polymers.

8. Method according to claim 7, wherein steps (b) and/or (c) is/are carried out in the presence of one or more amino acids selected from the group consisting of arginine, asparagine, glutamine, lysine and serine.

9. Method according to claim 8, wherein steps (b) and/or (c) is/are carried out in the presence of arginine.

10. Test strip for carrying out a method according to any of claims 4-9, wherein a C source which is fermented to acid by cariogenic bacteria and a pH indicator which changes colour upon acid formation are immobilized on the surface of the test strip in a way that upon contacting the test strip with a bacteria suspension the C source and the pH indicator are released from the test strip, and wherein the C source is selected from the group consisting of maltotriose, mannose, and N-acetylglucosamine, and wherein the cariogenic bacteria are selected from the group consisting of mutans streptococci and lactobacilli.

11. Test strip according to claim 10, wherein the test strip comprises a filter material having a pore size of less than 20 µm on which the C source and the pH indicator are immobilized.

12. Test strip according to claim 10 or 11, wherein the C source and/or the pH indicator have been immobilized on the test strip by means of a polymer lacquer.

13. Test kit for carrying out a method according to any of claims 4-9, comprising:
(a) a C source which is fermented to acid by the cariogenic bacteria, wherein the C source is selected from the group consisting of maltotriose, mannose, and N-acetylglucosamine, and wherein the cariogenic bacteria are selected from the group consisting of mutans streptococci and lactobacilli;
(b) a pH indicator that changes colour upon acid formation;
(c) a colour standard which allows for the semi-quantitative determination of the concentration of the cariogenic bacteria in the sample.

14. Test kit according to claim 13, wherein the colour standard is a multi-level standard.

15. Test kit according to claim 13 or 14, wherein the kit comprises at least one test strip according to any of claims 10-12.

## Revendications

1. Procédé d'évaluation semi-quantitative des bactéries cariogènes présentes dans un échantillon de plaque ou de salive, dans lequel procédé :
a) en option, on concentre les microorganismes présents dans un échantillon de plaque ou de salive ;
b) on met les microorganismes en contact avec une source de carbone qui est convertie en acide, par fermentation, par les bactéries cariogènes ;
c) on fait incuber les microorganismes et la source de carbone, dans des conditions qui permettent la formation sélective d'acide par les bactéries cariogènes ;
d) et l'on détermine le pH, au moins une fois dans le laps de temps de 12 heures après l'addition de la source de carbone ;
étant entendu
- que l'évaluation semi-quantitative des bactéries cariogènes présentes dans l'échantillon est réalisée par comparaison de la valeur du pH obtenue dans l'étape (d) avec au moins une valeur de référence,
- et que les bactéries cariogènes sont des streptocoques mutants et la source de carbone est du maltotriose.

2. Procédé d'évaluation semi-quantitative des bactéries cariogènes présentes dans un échantillon de plaque ou de salive, dans lequel procédé :
a) en option, on concentre les microorganismes présents dans un échantillon de plaque ou de salive ;
b) on met les microorganismes en contact avec une source de carbone qui est convertie en acide, par fermentation, par les bactéries cariogènes ;
c) on fait incuber les microorganismes et la source de carbone, dans des conditions qui permettent la formation sélective d'acide par les bactéries cariogènes ;
d) et l'on détermine le pH, au moins une fois dans le laps de temps de 12 heures après l'addition de la source de carbone ;
étant entendu
- que l'évaluation semi-quantitative des bactéries cariogènes présentes dans l'échantillon est réalisée par comparaison de la valeur du pH obtenue dans l'étape (d) avec au moins une valeur de référence,
- et que les bactéries cariogènes sont des lactobacilles et la source de carbone est choisie parmi du mannose et de la N-acétyl-glucosamine.

3. Procédé conforme à la revendication 1 ou 2, dans lequel, dans l'étape (d), on mesure le pH au moyen d'une électrode à pH.

4. Procédé conforme à la revendication 1 ou 2, dans lequel, dans l'étape (d), on évalue le pH au moyen d'un indicateur de pH dont la couleur change lors de la formation d'un acide.

5. Procédé conforme à la revendication 4, pour lequel l'indicateur de pH est choisi dans l'ensemble formé par les suivants : violet de bromocrésol, vert de bromocrésol, violet de méta-crésol, bleu de bromothymol, rouge de phénol et rouge de chlorophénol.

6. Procédé conforme à l'une des revendications précédentes, dans lequel on réalise l'étape (c) en présence d'un ou de plusieurs antibiotique(s) qui ne gêne(nt) pratiquement pas la croissance des bactéries cariogènes et/ou la formation d'acide par celles-ci.

7. Procédé conforme à l'une des revendications précédentes, dans lequel on réalise l'étape ou les étapes (b) et/ou (c) en présence d'un ou de plusieurs acide(s) aminé(s) et/ou d'un ou de plusieurs polymère(s) comportant un ou des acide(s) aminé(s).

8. Procédé conforme à la revendication 7, dans lequel on réalise l'étape ou les étapes (b) et/ou (c) en présence d'un ou de plusieurs acide(s) aminé(s) choisi(s) dans l'ensemble constitué par les arginine, asparagine, glutamine, lysine et sérine.

9. Procédé conforme à la revendication 8, dans lequel on réalise l'étape ou les étapes (b) et/ou (c) en présence d'arginine.

10. Bande de test conçue pour la mise en oeuvre d'un procédé conforme à l'une des revendications 4 à 9, à la surface de laquelle bande de test une source de carbone, qui est convertie en acide, par fermentation, par des bactéries cariogènes, et un indicateur de pH, dont la couleur change lors de la formation d'un acide, ont été immobilisés de telle manière que, quand la bande de test est mise en contact avec une suspension de bactéries, la source de carbone et l'indicateur de pH sont libérés de la bande de test, étant entendu que la source de carbone est choisie parmi du maltotriose, du mannose et de la N-acétyl-glucosamine et que les bactéries cariogènes sont choisies parmi des streptocoques mutants et des lactobacilles.

11. Bande de test conforme à la revendication 10, laquelle bande de test comprend un matériau filtre comportant des pores de taille inférieure à 20 µm, sur lequel la source de carbone et l'indicateur de pH ont été immobilisés.

12. Bande de test conforme à la revendication 10 ou 11, dans laquelle la source de carbone et/ou l'indicateur de pH ont été immobilisés sur la bande de test au moyen d'un vernis polymère.

13. Trousse de test conçue pour la mise en oeuvre d'un procédé conforme à l'une des revendications 4 à 9, qui comprend :
a) une source de carbone qui est convertie en acide, par fermentation, par des bactéries cariogènes, laquelle source de carbone est choisie parmi du maltotriose, du mannose et de la N-acétyl-glucosamine, et lesquelles bactéries cariogènes sont choisies parmi des streptocoques mutants et des lactobacilles,
b) un indicateur de pH dont la couleur change lors de la formation d'un acide,
c) et une échelle de couleurs, qui permet de faire une évaluation semi-quantitative de la concentration des bactéries cariogènes présentes dans l'échantillon.

14. Trousse de test conforme à la revendication 13, dans laquelle l'échelle de couleurs est une échelle à plusieurs degrés.

15. Trousse de test conforme à la revendication 13 ou 14, la quelle trousse comprend une bande de test conforme à l'une des revendications 10 à 12.
